(19) Europäisches Patentamt
European Patent Office
Office européen des brevets

(11) EP 3 979 251 A1

(12) EUROPEAN PATENT APPLICATION

(43) Date of publication:
06.04.2022 Bulletin 2022/14

(21) Application number: 20199741.8

(22) Date of filing: 02.10.2020

(51) International Patent Classification (IPC):
G16B 20/20 $^{(2019.01)}$     G16B 40/20 $^{(2019.01)}$

(52) Cooperative Patent Classification (CPC):
G16B 20/20; G16B 40/20

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Sophia Genetics S.A.
1025 Saint Sulpice (CH)

(72) Inventors:
• BIELER, Jonathan
1025 Saint-Sulpice (CH)

• POZZORINI, Christian
1025 Saint-Sulpice (CH)
• TUCK, Alex
1025 Saint-Sulpice (CH)
• XU, Zhenyu
1025 Saint-Sulpice (CH)

(74) Representative: Wenger, Joel-Théophile
IP Partners J. Wenger
Rte des Avouillons 6
1196 Gland (CH)

(54) METHODS FOR CHARACTERIZING THE LIMITATIONS OF DETECTING VARIANTS IN NEXT-GENERATION SEQUENCING WORKFLOWS

(57) A genomic data analyser may process the next generation sequencing data of a patient sample to identify whether a variant is present (positive variant calling), absent at a high confidence (negative variant calling), or equivocal (possible false negative calling) as falling under a calculated limit of detection (LOD). This LOD estimate corresponds the lowest variant allele fraction (VAF) detectable at the required sensitivity (true positive rate). The presently disclosed genomic data analyser may improve any legacy variant caller of the prior art by automatically calculating the limitations of variant calling detection for a user-defined sensitivity and minimal VAF of interest for any variant genomic position and/or mutation, depending on analytical factors of the NGS assay and workflow such as the sample type, the DNA sample amount and the NGS assay library conversion rate (LCR), and/or its molecular barcoding capability, as well as its NGS assay error profile.

Figure 2

**Description**

FIELD OF THE INVENTION

Methods described herein relate to genomic analysis in general, and more specifically to the use of genomic information for detecting and characterizing genomic variants.

BACKGROUND OF THE INVENTION

Next-generation sequencing (NGS) assays are central to many branches of precision medicine, including liquid biopsies, tumour profiling and immunotherapy. However, translation to the clinic is impeded by reproducibility issues arising from unavoidable technical limitations. Regulatory and professional bodies are therefore calling for urgent measures to report NGS limitations so that errors are foreseen.

Clinical next-generation sequencing (NGS) assays present great opportunities for precision medicine as they can screen thousands of genomic loci for disease relevant alterations. Indeed, we are in the midst of a boom in which NGS technologies are being developed for diverse applications including pan-cancer profiling, liquid biopsies, biomarker discovery, immune profiling and personalised cancer vaccines *(Chuah & Chew, 2020, J. Immunother. cancer 8, e000363; Zviran et al., 2020, Nat. Med. 26(7):1114-1124; Malone et al., 2020, Genome Med. 12, 8).* Despite their huge potential, recent studies found that NGS assays suffer from significant reproducibility issues *(Stetson et al., 2019, JCO Precis. Oncol. 1-9; Paweletz et al., 2019, JCO Precis. Oncol. 1-3; Kuderer et al., 2017, JAMA Oncol. 3, 996-998).* This is caused by incorrect calls (i.e. false positives and false negatives) occurring unexpectedly often, with a large-scale evaluation of NGS databases putting the false negative rate at 40-45% *(Kim et al., 2019, PLoS One 14, e0222535).* This problem is particularly acute for NGS technologies encountering low frequency genomic alterations, as is the case with early or non-invasive disease monitoring. For example, circulating tumour DNA (ctDNA) profiling is a minimally invasive approach capturing the heterogeneous and evolving nature of tumours *(Heitzer et al., 2019, Nat. Rev. Genet. 20)* that relies on detecting variants down to 0.1-0.3% *(Matsumoto et al., 2020, Lung Cancer 139, 80-88; Jiang et al., 2019, Mol Med Rep 20, 593-603).* These low variant allele frequencies (or equivalently, variant allele fractions) (VAFs) arise because ctDNA accounts for only a minority of cell-free DNA (cfDNA) in blood *(Heitzer et al., 2020, Trends Mol. Med. 26, 519-528; Sun et al., 2015, PNAS 112, E5503 LP-E5512).* Despite claims that NGS assays can detect VAFs of <1% *(Paweletz et al., 2019, supra;* Chien et al., 2017, J. Clin. Oncol. 35, e23065-e23065; Plagnol et al., 2018, PLoS One 13, e0193802), a recent study found that when identical ctDNA samples were sent to different commercial laboratories, up to 90% of the low VAF variants were overlooked. This resulted in a high discordance between the results obtained by the different laboratories *(Stetson et al., 2019, supra).*

These reproducibility issues and high false negative rates are a major problem for clinical and research communities, as they undermine confidence in new technologies, cloud the results of clinical trials and can ultimately lead to incorrect treatment decisions. Discrepancies between NGS assays are often attributed to biological factors such as tumour heterogeneity *(Kuderer et al., 2017, supra).* However, *Stetson et al.* showed that most testing errors arise from technical factors such as background noise or assay-specific nucleotide biases *(Stetson et al., 2019, supra).* This revelation recently sparked intense debate among clinicians, the FDA and other professional bodies (*U.S. Food and Drug Administration, 13 April 2018, Considerations for Design, Development, and Analytical Validation of Next Generation Sequencing-Based In Vitro Diagnostics Intended to Aim in the Diagnosis of Suspected Germline Diseases; Weber et al., 2020, Cancers (Basel). 12, 1588),* as it shows we have grossly underestimated the influence of technical factors and are unknowingly exceeding the capabilities of NGS assays. It also implies that addressing these technical hurdles can dramatically improve the reliability of NGS testing, and there is now an urgent need to do just this.

An obvious solution is to develop NGS assays with fewer technical limitations and, as detecting a genomic alteration relies on distinguishing signal from noise, research efforts have targeted both of these elements. For example, digital error suppression and barcoding strategies dramatically reduce background noise, while amplicon- or capture-based enrichment boosts signal across specific genomic regions *(Newman et al., 2016, Nat. Biotechnol. 34, 547-555).* Despite tremendous progress, there are some fundamental limitations that cannot be overcome, such as how much patient material can be sampled or stochastic sampling noise. Furthermore, for applications like early disease detection, there will always be a need to detect the lowest possible VAFs, so NGS assays will continually be pushed to their limits. Therefore, although NGS testing errors can be reduced, they cannot be eliminated, meaning that assay improvements are only part of the solution. Recognising this, regulatory and professional bodies, clinicians and research communities are demanding that assay limitations are accurately and transparently reported alongside NGS results (U.S. Food and Drug Administration, 13 April 2018, *Considerations for Design, Development, and Analytical Validation of Next Generation Sequencing-Based In Vitro Diagnostics Intended to Aim in the Diagnosis of Suspected Germline Diseases;* Merker et al., 2018, J. Clin. Oncol. 36, 1631-1641; Tack et al., 2018, J. Mol. Diagnostics 20, 743-753). The idea is not to eliminate testing errors, but to foresee them and act accordingly. At the same time, this increases confidence in the remaining results. Together with assay improvements, this elegant solution could eliminate most incorrect results.

Essentially, this strategy requires to evaluate the likelihood that each positive or negative call is correct. For positive calls this is relatively straightforward, as sources of errors are well understood, and positive calls usually limited. Retesting is therefore feasible for all positive results deemed to be ambiguous, even if we are overly conservative. The situation is more complex for negative calls, which usually run into the thousands. Ideally, for each "negative" position the limit of detection (LOD) may be reported. This is the lowest VAF detectable at the required sensitivity (true positive rate). Knowing the LODs would reveal sites where a variant might not have been detected due to technical limitations. These potential false negatives could then be retested, focusing on hotspots for actionable mutations. Unfortunately, there is currently no effective way to calculate the LOD for every position interrogated by an NGS assay. This is because experimental approaches are limited to a handful of variants (leading to the practise of reporting a global LOD), and in silico predictions are held back by a lack of understanding of how LOD is determined (likely by many variables acting in combination). Previous modelling approaches either omit some variables, are limited to specific assays, require specialised controls, or are not integrated into a full bioinformatic workflow (Blomquist et al., 2015, Biomol. Detect. Quantif. 5, 30-37; Petrackova et al., 2019, Front. Oncol. 9, 1-6; Ma et al., 2019, Genome Biol. 20, 50; Xu et al., 2017, BMC Genomics 18, 5). An accurate and universal method to determine LOD and report it alongside NGS results is therefore a key unresolved challenge that must be overcome to improve the reliability and reproducibility of genetic testing.

In summary, there is a critical need to integrate the identification, characterization and reporting of NGS assay limitations into NGS workflows routinely used in clinical practise and research. This is key to improving the reliability of NGS testing, as it is now clear that incorrect calls (i.e. false positives and false negatives) are a persistent issue that cannot be resolved solely by technological improvements to NGS assays (which has been the focus of most prior art approaches). All NGS technologies have technical limitations, and there may always remain applications in which NGS technologies must operate near their limits. Thus, improved identification and reporting of assay limitations may be a way to satisfactorily address the incorrect variant calls that will inevitably occur. This capability needs to be integrated into automated NGS workflows that are cost effective, easy to operate, and suitable for routine clinical practice. Such workflows should be able to process data from different patient sample types, NGS technologies and variant types, account for the different sequencing error profiles that will be encountered, and operate in line with the requirements of the end user (e.g. required variant calling sensitivity). Furthermore, as the range of NGS technologies in routine use expands, these limitation-aware workflows must be easily adjustable and continue to meet the requirements of the assay and end user (e.g. the range of VAFs encountered). Preferably, initial setup would involve a consistent, robust and straightforward process, and the end user would not need to understand the intricacies of the NGS assay(s) and computational analyses. By identifying and reporting assay limitations, these workflows would enable the end user to make an informed decision as to whether further genomic analysis is required, and if so, for which genomic positions and variant types.

BRIEF SUMMARY

A method is proposed for estimating a limitation of detecting a nucleic acid sequence variant (*chr,pos,alt,rej*) in data generated by a next-generation-sequencing assay from a patient sample, the method comprising obtaining alignment data, relative to a reference genome, from the patient sample NGS data; identifying from the alignment data, with a variant caller, that said variant does not have a positive call status; obtaining a measurement of the molecular count for the patient sample at the genomic position (*chr,pos*) of said variant; obtaining one or more analytical factors of the NGS assay used to process the patient sample; producing, with a statistical model, synthetic alignment data for one or more simulated VAFs as a function of the measured molecular count and the analytical factors of the NGS assay; and estimating, from the synthetic alignment data, the detection sensitivity limitation of said variant caller as a function of one or more of the simulated VAFs, for said assay, said DNA sample and said variant (*chr, pos, ref, alt*). The synthetic data may be one or more BAM files, or one or more sets of different NGS data alignment features, for each simulated VAF value. The statistical model may be a machine learning generative model or a biophysical generative model.

In a possible embodiment, the method may further comprise obtaining a user-defined minimal variant allele fraction of interest (mVAF) for said variant; obtaining a user-defined desired sensitivity for calling said variant as positive; estimating, from the estimated detection sensitivity function, the sensitivity for said mVAF; and classifying the variant status as negative or equivocal as a function of said desired and estimated sensitivity; and reporting the variant status to an end user.

In a possible embodiment, the method may further comprise obtaining a user-defined desired sensitivity for calling said variant as positive, estimating, from the estimated detection sensitivity function, a limit of detection $LOD_{est}$ value as the lowest simulated VAF detectable with a sensitivity larger or equal to the user-defined sensitivity, and reporting the estimated limit of detection $LOD_{est}$ value to the user.

In a possible embodiment, the method may comprise obtaining a user-defined minimal variant allele fraction of interest (mVAF) for said variant; obtaining a user-defined desired sensitivity for calling said variant as positive; estimating, from the estimated detection sensitivity function, a limit of detection $LOD_{est}$ value as the lowest simulated VAF detectable with a sensitivity larger or equal to the user-defined sensitivity; and if the m VAF for said variant is larger or equal to the $LOD_{est}$, classifying the variant status as negative, otherwise classifying the variant status as equivocal; and reporting the variant status and the $LOD_{est}$ value to an end-user.

In a possible embodiment the method may estimate the molecular count in the NGS sequencing data according to molecular identifiers measurements in the alignment data.

In a possible embodiment, the method may comprise measuring, in the alignment data, the total coverage at the genomic position *(chr,pos)* of said variant and producing, with the statistical model, the synthetic alignment data for one or more simulated VAFs as a function of the coverage.

In a possible embodiment, the method may comprise obtaining a DNA sample amount measurement and estimating the molecular count in the library as a function of the DNA sample amount and the LCR value for the genomic position *(chr; pos)* in the LCR profile. In a possible embodiment, the LCR profile may be a constant value for all genomic positions. In an alternate embodiment, the LCR profile may be a table of the library conversion rate value at each genomic position, or for a set of genomic positions. In a possible embodiment, the LCR profile may depend on the DNA sample type.

In a possible embodiment, the analytical features of the NGS assay may comprise an NGS assay error profile as a constant value for all variants, or as a table of the error rate value at each variant position *(chr,pos)* or set of positions, or as a table of the error rate value for each variant mutation type *(alt,ref)* or set of variant mutations, or as a table of the error rate value for each variant *(chr,post,ref,alt)*. In a possible embodiment, the NGS workflow error profile may depend on a DNA sample type.

BRIEF DESCRIPTION OF THE DRAWINGS

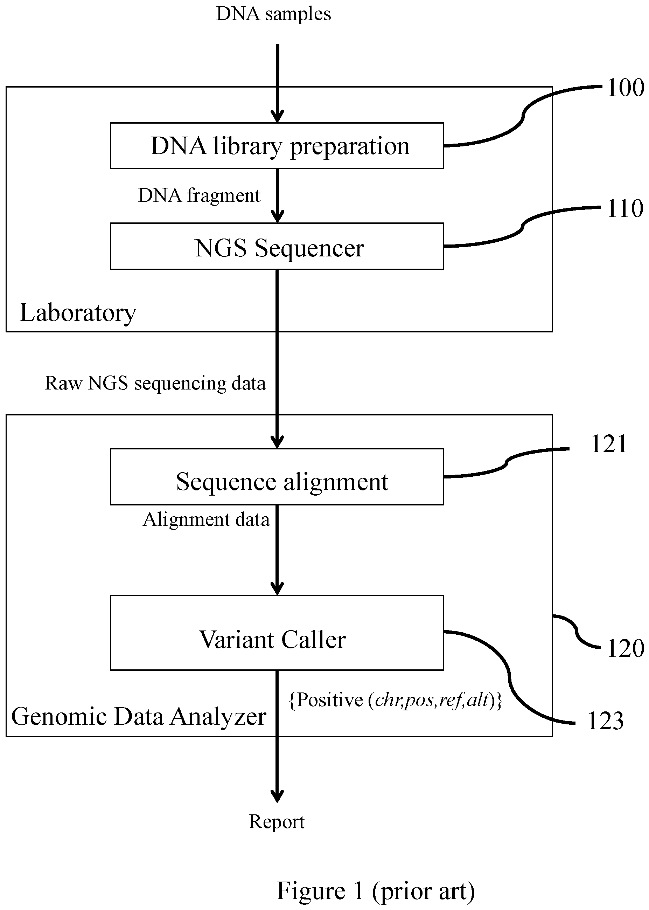

**Figure 1** represents a next generation sequencing system according to certain embodiments of the present disclosure.

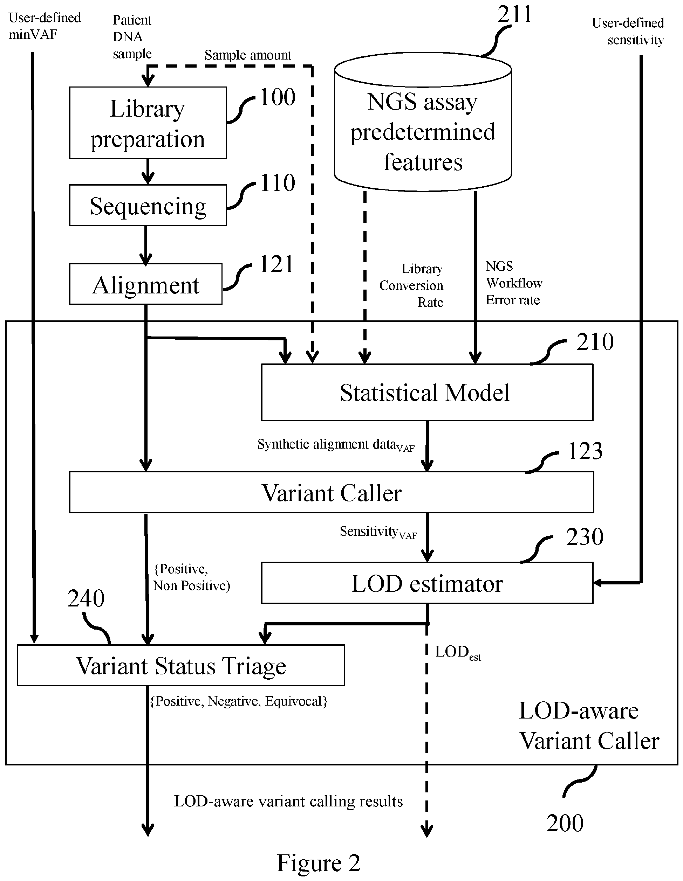

**Figure 2** represents an improved genomic analysis system according to certain embodiments of the present disclosure.

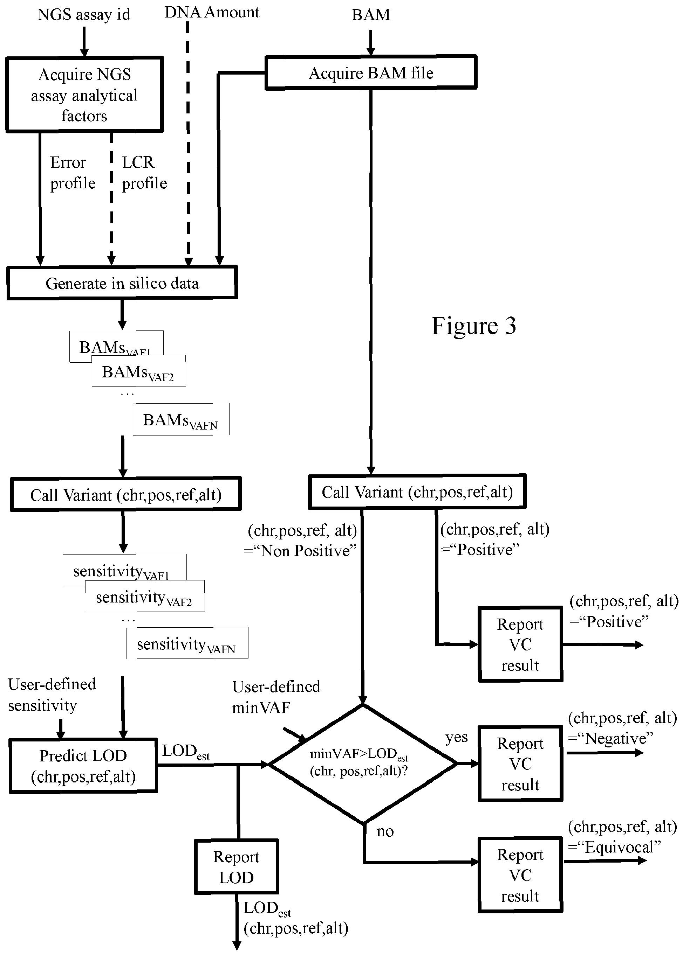

**Figure 3** represents an improved variant calling workflow according to certain embodiments of the present disclosure.

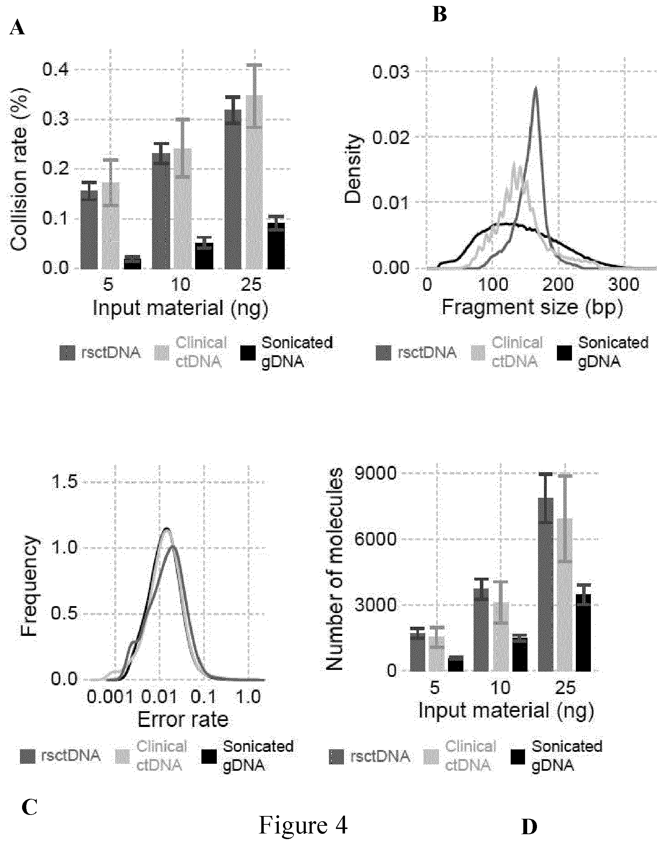

**Figure 4** shows comparison of ctDNA reference materials as detailed in Example 1. Commutability of sonicated gDNA and rsctDNA, with clinical ctDNA, was assessed using assay A3. **A:** Collision rate, defined as the number of mapping positions at which more than one molecule (defined using molecular barcodes) aligned divided by the total number of mapping positions occupied in the data, plotted for each input type and amount. Error bars indicate the standard deviation across different genomic regions. **B:** Fragment size distribution. **C:** Distribution of error rates at genomic positions (n=6400) targeted by all assays. **D:** Average number of unique molecules covering each interrogated genomic position. Error bars indicate the standard deviation across different genomic regions.

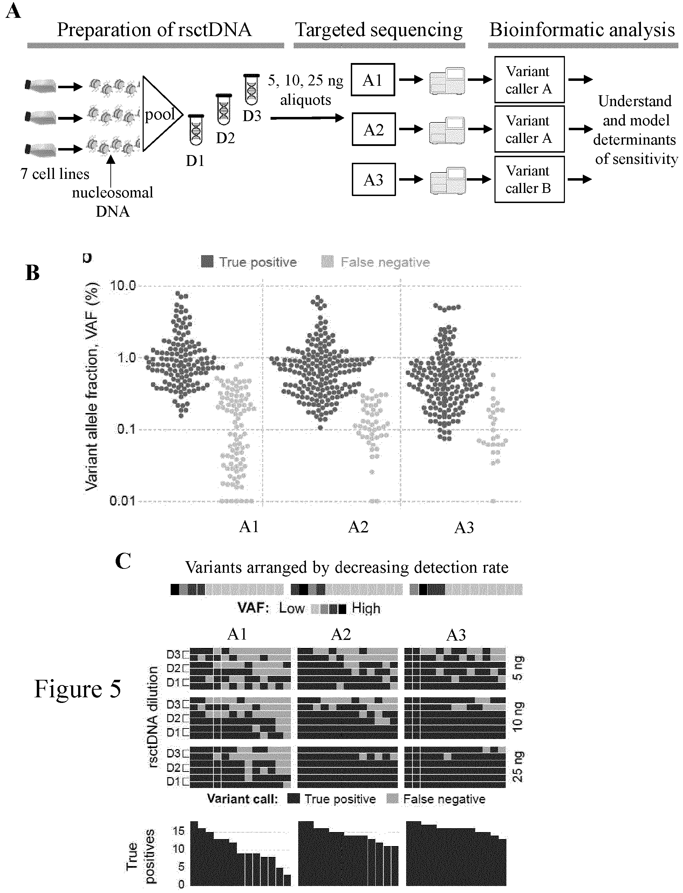

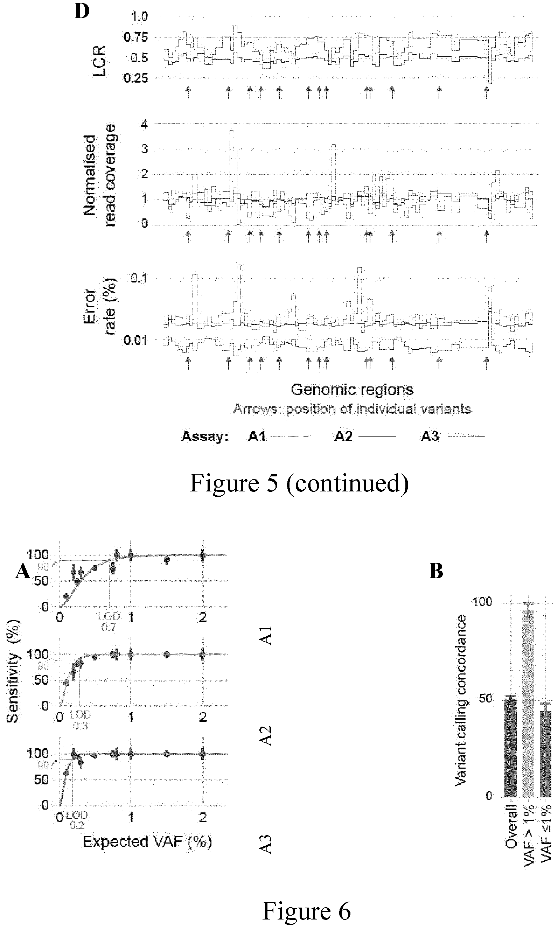

**Figure 5** shows that LOD is determined at multiple scales. **A:** Experimental design as described in Example 1. **B:** True positive and false negative calls and measured VAFs from the experiment shown in A. In the absence of signal, VAF is set to 0.01%. **C:** Individual variant calls from 'A' arranged by VAF (individual columns), NGS assay (vertical groups), input material amount (horizontal groups) and rsctDNA dilutions (pairs of rows). The VAF for individual variants is indicated by the grey boxes of various shades ("VAF"), and for each NGS assay, variants are sorted by the number of true positives (which are summed in the lower plots). **D:** Library conversion rate (top), relative sequencing coverage (middle) and background noise (bottom) of each NGS assay shown as a function of genomic region. Arrows show the genomic positions of the 13 confirmed variants in 'C'. For visualisation purposes, data were averaged within exons. Relative sequencing coverage was calculated by averaging the mean-normalized coverage profiles observed in multiple samples. Error rate profiles show the mean of the statistical distribution (A1-A2: beta-binomial, A3: binomial) fitted to the number of artefactual base calls observed in multiple samples.

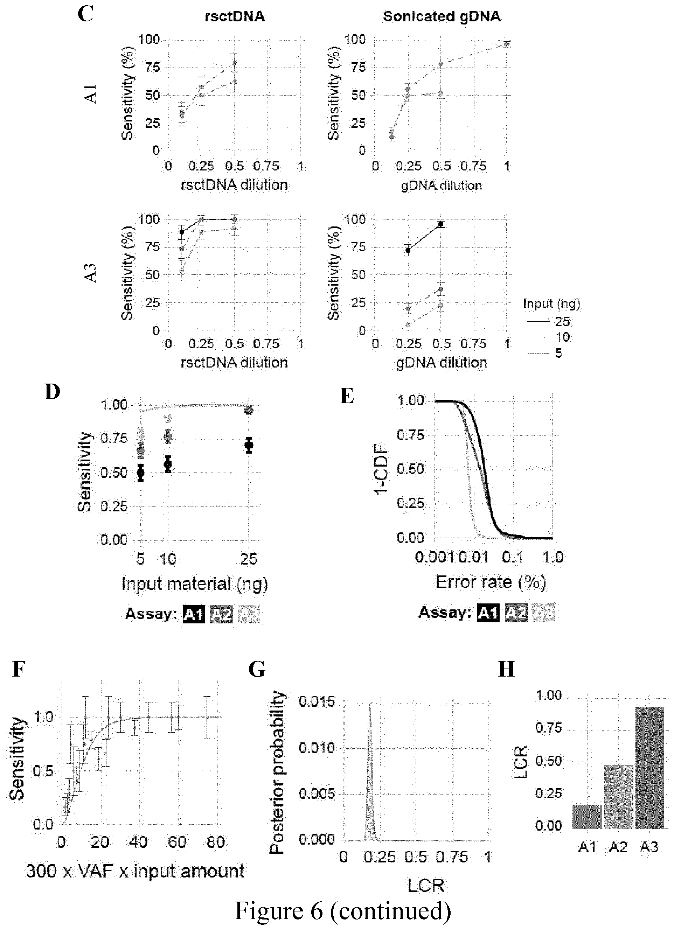

**Figure 6** shows investigating factors that determine variant calling sensitivity. **A:** Sensitivity (true positive count / (true positive count + false negative count)) measured for each NGS assay as a function of expected VAF. For each assay, an overall LOD (vertical line) was measured by fitting the data with a sigmoidal function (solid line), requiring a sensitivity of >90%. **B:** Variant calling concordance for fig. 5B, defined as the fraction of confirmed SNVs having the same variant calling status in all NGS assays. The three bars show the concordance computed using all confirmed SNVs (n=234), SNVs with VAF >1% (n=30) and SNVs with VAF <1% (n=204). **C:** Sensitivity of NGS assays A1 and A3 when using various dilutions of rsctDNA (left) or sonicated gDNA (right). **D:** Sensitivity of each NGS assay plotted against input rsctDNA amount, using the data presented in fig. 5. Error bars represent one standard deviation of theoretical uncertainty given the number of variants used to measure sensitivity. The light grey line shows the maximum theoretical sensitivity given the amount of starting material used. **E:** Fraction of genomic positions with error rates greater than or equal to a specific level (x-axis) measured for each NGS assay. **F** and **G:** Inferring the library conversion rate of NGS Assay A1 from variant calling results. **F:** The variant calling results shown in Fig. 5 were used to measure sensitivity (grey dots) as a function of the expected number of mutated DNA molecules present in the original sample. Error bars represent one standard deviation of theoretical uncertainty expected given the finite number of variants used to measure sensitivity. Data were fitted with a theoretical model (grey line) describing sensitivity as a function of the number of mutated molecules expected in the original sample and library conversion rate. The grey line shows the theoretical sensitivity computed using the maximum a posteriori estimation of library conversion rate **G:** Posterior distribution of library conversion rate obtained by fitting a theoretical model to the data in F. **H:** Library conversion rate estimation for the NGS assays, calculated by dividing the observed number of molecules in the NGS data presented in fig. 5 by the number expected given the amount of input DNA used (assays A2 and A3) or using the approach shown in F and G (assay A1).

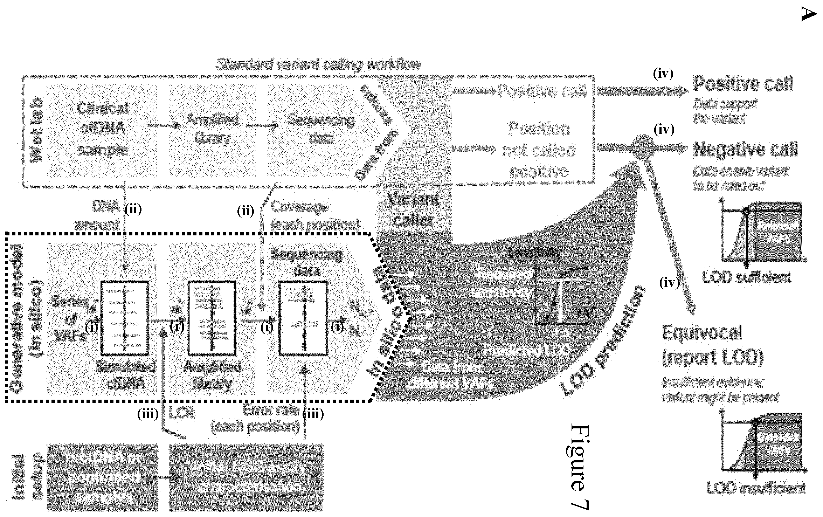

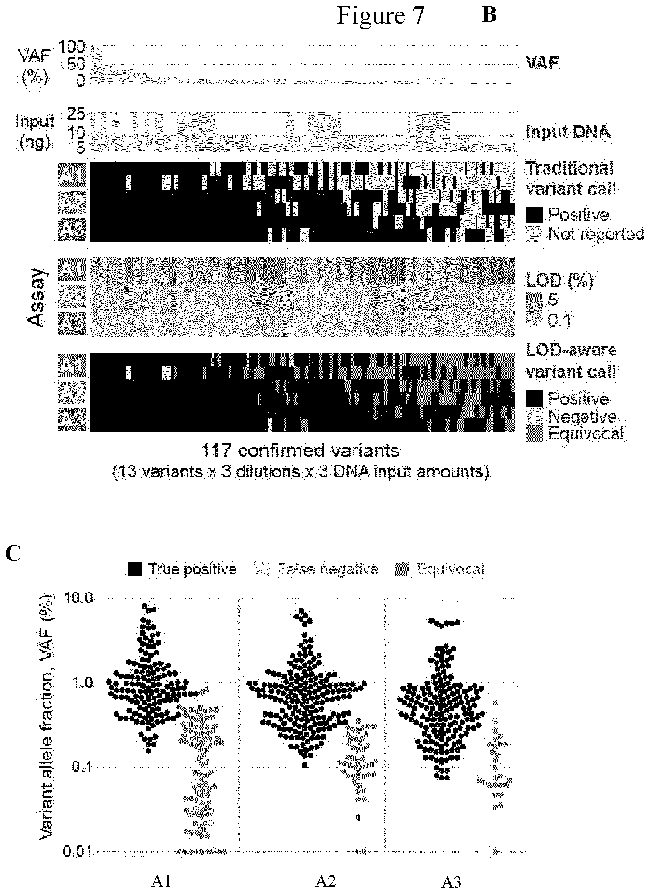

**Figure 7** shows LOD-aware variant calling framework. **A:** Overview of the LOD-aware variant calling framework, highlighting how it enhanced the standard variant calling approach. In the standard approach (top track, boxed), NGS libraries obtained from clinical cfDNA samples are sequenced, then the data are interpreted by a variant caller which outputs positive calls. Positions where variants are not called are not usually reported. The LOD-aware framework built on this approach by using an *in silico* statistical model (centre, encapsulated in thick dashed lines) to predict LODs for every interrogated position (right, curved block arrow) that can be reported alongside all results (arrows marked (iv)). The model required several inputs. These included the LCR and an error model, which were calculated once when setting up a given assay (bottom, arrows marked (iii)). Additional model inputs were obtained for each sample, including the amount (mass, in nanograms) of DNA used and sequencing coverage, with the latter being measured from the NGS data (top, arrows marked (ii)). Note that sequencing coverage and error rate were defined for each genomic position. After receiving these inputs, the model (arrows marked (i)) simulated a dilution experiment for each variant of interest by producing synthetic NGS data as follows. First, the model used the input DNA amount to estimate the number of ctDNA molecules covering a given genomic locus. Then, for a range of VAFs, the entire NGS workflow was simulated, including library preparation (ctDNA molecules were incorporated according to the LCR), PCR amplification and sequencing (reads were sampled to the measured sequencing depth, and errors injected based on the measured error rate). Short horizontal grey lines represent DNA fragments in the sample, amplified library and sequencing data. Black rectangles and grey triangles represent DNA mutations (at the position indicated by the vertical dashed line) and artefactual base calls, respectively. The model accounted for sampling variability (coin flip icons) during DNA sample generation, library preparation and sequencing. The output from the model (a series of VAFs and ref (N) and alt ($N_{ALT}$) read counts) was then presented to the variant caller (light grey), which called variants for each simulated read set and constructed a curve of estimated sensitivity as a function of VAF (dark grey curved block arrow). The predicted LOD could then be read off of this curve and was defined as the lowest VAF that could be detected with a desired sensitivity. Finally, the set of predicted LODs could be used to classify positions at which no variant was detected as either negative (the LOD is sufficiently low that a variant would have been detected if present) or equivocal (a false negative cannot be ruled out). **B:** The output obtained by performing LOD-aware variant calling on the rsctDNA dataset in fig. 5. The observed VAF, input amount, variant call using a standard approach, predicted LOD and LOD-aware variant call is shown (top to bottom) for each confirmed variant, dilution and input amount (columns). Each pair of consecutive rows shows the results of replicates tested with NGS assays A1, A2 and A3, and variants are sorted by confirmed VAF and input amount. LOD-aware variant calls are defined as follows: positive - the variant is identified by the variant caller, negative - the variant is not identified by the variant caller and the VAF of interest is greater than or equal to the predicted LOD, equivocal - the variant is not identified by the variant caller and the VAF of interest is lower than the LOD. The "VAF of interest" is defined as that determined by dPCR, which was used to mimic clinical insight into the minimum expected VAF. **C:** LOD-aware variant calling status and measured VAF for each variant present in the rsctDNA samples. Note that "negative" calls are labelled as "false negative", as in these cases, a confirmed variant was not detected even though the predicted LOD is sufficient.

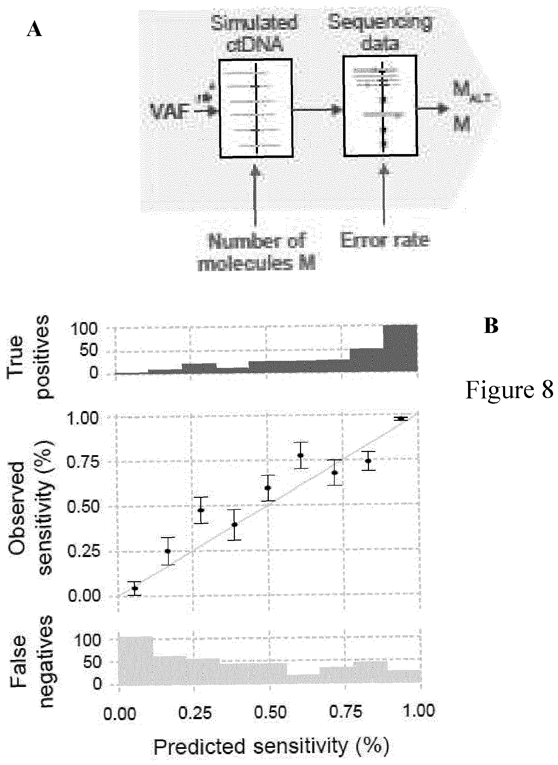

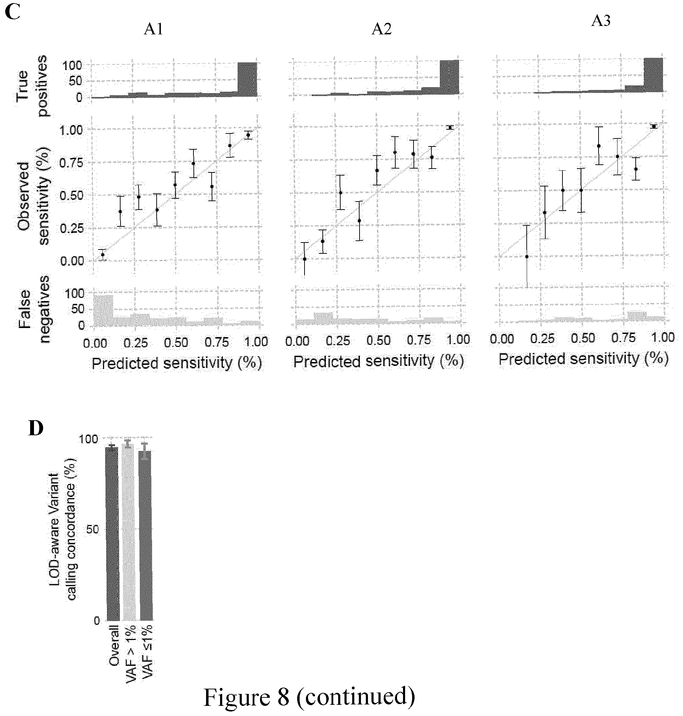

**Figure 8** shows validation of the LOD-aware variant calling framework for rsctDNA samples. **A:** Schematic representation of the generative model used for NGS assay A3. For a given VAF and observed number of duplex molecules M, the statistical model described the number of molecules supporting the variant $M_{ALT}$. Grey lines represent DNA fragments in the sample and sequencing data. Black rectangles and grey triangles represent DNA mutations and artefactual base calls, respectively. **B** and **C:** Variant calls shown in figure 7B are grouped by predicted sensitivity (x-axis), and the true positive (TP) count, false negative (FN) count and sensitivity (TP/(TP + FN)) plotted for each group. Error bars indicate one standard deviation of theoretical uncertainty given the number of variants used to measure sensitivity. The diagonal grey line shows the ideal case, where observed sensitivity is equal to predicted sensitivity. **B** shows the results aggregated for all NGS assays, whereas **C** shows each assay individually. **D:** Variant calling concordance between the three NGS assays, based on figure 7C and excluding equivocal calls from the calculation.

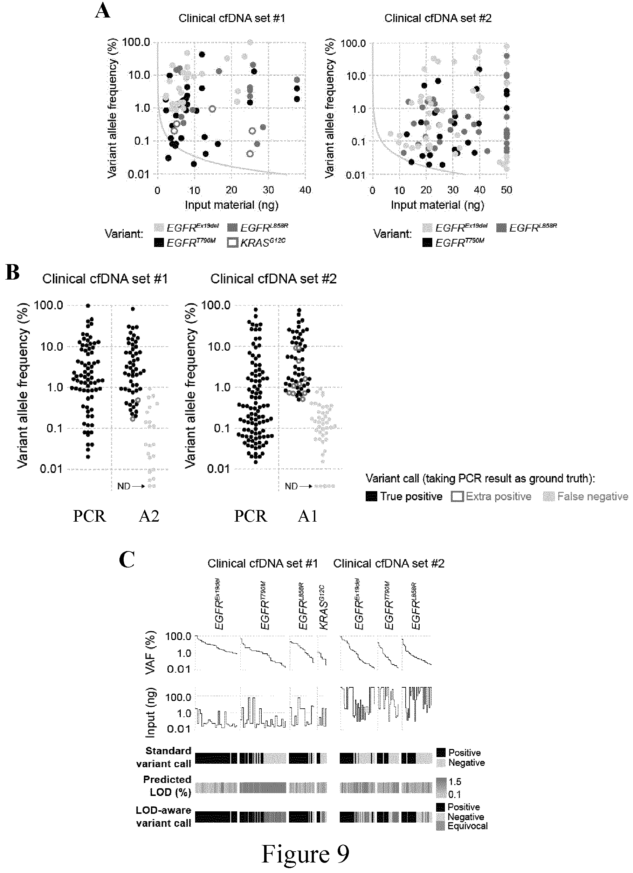

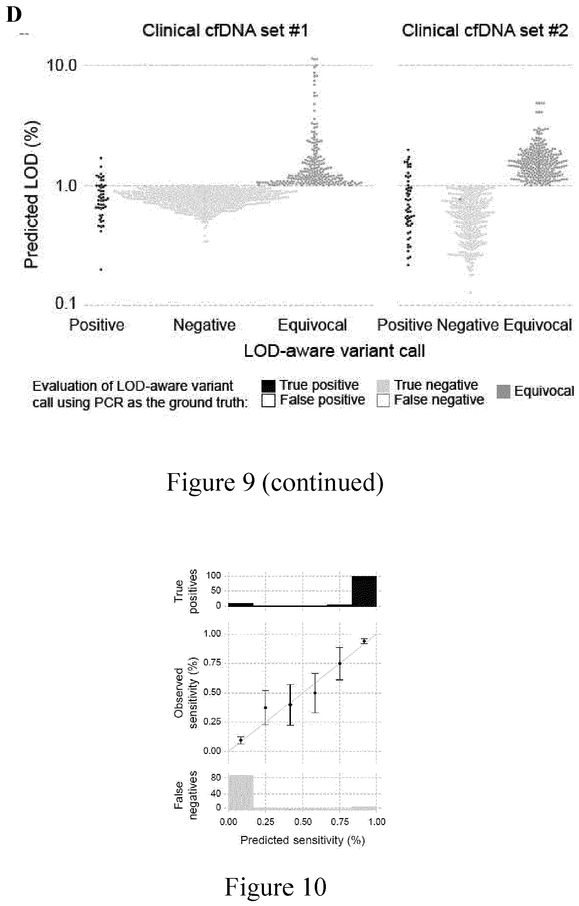

**Figure 9** shows LOD-aware variant calling for 580 clinical cfDNA samples. **A:** VAF and input material amount for variants identified using dPCR (ddPCR and BEAMing) for two sets of clinical NSCLC cfDNA samples. Points are coloured by the variant that they correspond to. The light grey line corresponds to VAFs and input materials for which, assuming an LCR of 100%, one molecule supporting the variant is expected. **B:** Variant calling status and dPCR and NGS VAF measurements for the confirmed variants from 'A' after testing using NGS assay A1 (for set #1) and A2 (for set #2). Extra positives are variants detected by NGS but not dPCR, and variants not detected (ND) by NGS are indicated with an arrow (and placed at the bottom edge of the plot). **C:** Output obtained by performing LOD-aware variant calling on the clinical datasets. The observed VAF, input amount, variant call using a standard approach, predicted LOD, and LOD-aware variant call are shown for each confirmed variant. LOD-aware variant calls were defined using 1% as the "minimal VAF of interest" (i.e. the lowest VAF we need to reliably detect). Using this 1% threshold, divided were non-positive positions into "negative" (LOD<1%) and "equivocal" (LOD>1%) classes. **D:** LOD prediction and LOD-aware variant calling status evaluated by comparing to the ground truth (dPCR status). Here included are dPCR-confirmed variants (shown in 'C') as well as all dPCR-validated negative cases for the four tested positions in the 580 samples. Positions with an LOD-aware "positive" label are classed as "true positive" (TP) when there is a dPCR-confirmed variant, and "false positive" (FP) when there is not. Positions with an LOD-aware "negative" label are classed as "true negative" (TN) if no variant was detected by dPCR (or a variant was detected by dPCR with VAF<1%), and "false negative" (FN) if there is a dPCR-confirmed variant with VAF>1%. Equivocal (E) calls retain the label "equivocal" and are positions we would exclude from subsequent analyses due to assay insufficiencies. For clinical cfDNA set #1, n=924, TP=44, FP=4, TN=607, FN=0, E=269, concordance=99.4%. For clinical cfDNA set #2, n=696, TP=53, FP=2, TN=352, FN=1, E=288, concordance=99.3%. Combined concordance (set #1 and #2) = 99.3%.

**Figure 10** shows validation of the sensitivity predicted by LOD-aware variant calling for clinical samples. Data shown in fig. 9C were analysed to verify the agreement between predicted and observed sensitivity (black dots). Variant calls were grouped according to their predicted sensitivity. For each group, observed sensitivity (black dots) was computed by counting the number of true positives (TP count, top histogram) and false negatives (FN count, bottom). The error bars indicate the standard deviation of the posterior distribution of the observed sensitivities.

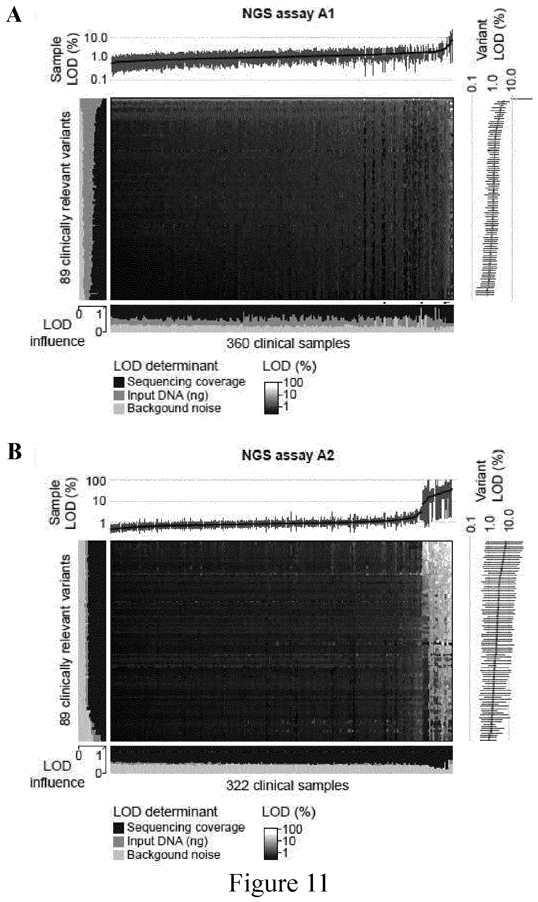

**Figure 11** shows the LOD landscape for clinically relevant variants. **A:** Predicted LODs for 89 variants annotated as pathogenic in ClinVar are displayed on a heatmap, with each column corresponding to one clinical sample tested with NGS assay A1 (n=360). Essentially, this demonstrates the lowest variant fraction that could be detected at each site in each sample, thus revealing our potential to identify variants. Rows and columns are sorted according to the average LOD computed across samples and variants, respectively. Top: the average LOD for each clinical sample, across all variants. Right: the average LOD for each variant, across all samples. Error bars indicate 5% and 95% quantiles. Left and bottom: "LOD influence" is an analysis to determine which of the three model inputs (background noise, coverage depth or input amount) has the most influence on LOD, and thereby which could be adjusted to improve the LOD. This is evaluated by calculating the increase in predicted sensitivity (at the current LOD) when one of the three model inputs is adjusted (improved) by 10%. The values associated with changing each input are averaged across samples (left) or variants (bottom), then normalised by dividing by the sum of the three average values (as described in *Sensitivity analysis*). Thus, the length of each coloured line represents the relative gain in sensitivity that would be achieved by improving each of the input factors. **B:** Predicted LODs, limiting factors and summary statistics for the second set of clinical cfDNA samples, processed with NGS assay A2. Data are presented as in 'A'.

DETAILED DESCRIPTION

The present disclosure is based, at least in part, on the discovery that the presently disclosed genomic data analyser may process the next generation sequencing data of a patient DNA sample to identify whether a variant is present (positive variant calling), absent at a high confidence (negative variant calling), or equivocal (possible false negative calling) as falling under a calculated limit of detection (LOD). This LOD estimate corresponds to the lowest variant allele fraction (VAF) detectable at the required sensitivity (true positive rate). The presently disclosed genomic data analyser may improve any legacy variant caller of the prior art by automatically accounting for the limitations of variant calling detection for a user-defined sensitivity and minimal VAF of interest for any variant genomic position and/or mutation depending on analytical factors of the NGS assay and workflow such as the sample type; the DNA sample amount and the NGS assay library conversion rate (LCR), or its molecular barcoding capability; as well as its NGS assay error profile.

Definitions

A **"DNA sample"** refers to a nucleic acid sample derived from an organism, as may be extracted for instance from a solid tumour or a fluid. The organism may be a human, an animal, a plant, a fungus, or a microorganism. The nucleic acids may be found in a solid sample such as a Formalin-Fixed Paraffin-Embedded (FFPE) sample. Alternately, the nucleic acids may be found in a liquid biopsy, possibly in limited quantity or low concentration, such as for instance circulating tumour DNA in blood or plasma.

A **"DNA fragment"** refers to a short piece of DNA resulting from the fragmentation of high molecular weight DNA. Fragmentation may have occurred naturally in the sample organism, or may have been produced artificially from a DNA fragmenting method applied to a DNA sample, for instance by mechanical shearing, sonication, enzymatic fragmentation and other methods. After fragmentation, the DNA pieces may be end repaired to ensure that each molecule possesses blunt ends. To improve ligation efficiency, an adenine may be added to each of the 3' blunt ends of the fragmented DNA, enabling DNA fragments to be ligated to adaptors with complementary dT-overhangs.

A **"DNA product"** refers to an engineered piece of DNA resulting from manipulating, extending, ligating, duplicating, amplifying, copying, editing and/or cutting a DNA fragment to adapt it to a next-generation sequencing workflow.

A **"DNA-adaptor product"** refers to a DNA product resulting from combining a DNA fragment with a DNA adaptor to make it compatible with a next-generation sequencing workflow. Different approaches exist to combine a DNA fragment and a DNA adaptor. These includes amplicon-based protocols or capture based protocols, or hybrid protocols.

A **"DNA library"** refers to a collection of DNA products or DNA-adaptor products that adapt DNA fragments for compatibility with a next-generation sequencing workflow.

A **"DNA amount"** refers to the quantity of purified DNA present in the sample that is processed with a NGS assay. DNA amount is usually measured in nanograms or micrograms. DNA amount can also be measured in units of human genome equivalents or haploid human genome equivalents.

A **"nucleotide sequence"** or a **"polynucleotide sequence"** refers to any polymer or oligomer of nucleotides such as cytosine (represented by the C letter in the sequence string), thymine (represented by the T letter in the sequence string), adenine (represented by the A letter in the sequence string), guanine (represented by the G letter in the sequence string) and uracil (represented by the U letter in the sequence string). It may be DNA or RNA, or a combination thereof. It may be found permanently or temporarily in a single-stranded or a double-stranded form. Unless otherwise indicated, nucleic acids sequences are written left to right in 5' to 3' orientation.

**"Ligation"** refers to the joining of separate double stranded DNA sequences. The latter DNA molecules may be blunt ended or may have compatible overhangs to facilitate their ligation. Ligation may be produced by various methods, for instance using a ligase enzyme, performing chemical ligation, and other methods.

**"Amplification"** refers to a polynucleotide amplification reaction to produce multiple polynucleotide sequences replicated from one or more parent sequences. Amplification may be produced by various methods, for instance a polymerase chain reaction (PCR), a linear polymerase chain reaction, a nucleic acid sequence-based amplification, rolling circle amplification, and other methods.

A **"Library Conversion Rate"** (LCR) refers to the percentage of input DNA molecules covering a genomic region of interest present in a DNA sample, which an NGS assay can successfully transform into sequenceable DNA products. In capture-based NGS assays, LCR can also refer to the percentage of input DNA molecules present in a DNA sample, for which at least one sequenceable DNA product was obtained after completion of the capture step.

**"Sequencing"** refers to reading a sequence of nucleotides as a string. High throughput sequencing (HTS) or next-generation-sequencing (NGS) refers to real time sequencing of multiple sequences in parallel, typically between 50 and a few thousand base pairs per sequence. Exemplary NGS technologies include those from Illumina, Ion Torrent Systems, Oxford Nanopore Technologies, Complete Genomics, Pacific Biosciences, BGI, and others. Depending on the actual technology, NGS sequencing may require sample preparation with sequencing adaptors or primers to facilitate further sequencing steps, as well as amplification steps so that multiple instances of a single parent molecule are sequenced, for instance with PCR amplification prior to delivery to flow cell in the case of sequencing by synthesis. In whole genome sequencing (WGS), DNA fragments originating from all genomic regions are sequenced. Depending on the NGS assay, only DNA fragments originating form one or more specific regions of interest (e.g., a particular gene of interest or all the exons present in the genome) are enriched and successfully sequenced. Enrichment can be performed using different technologies, including capture sequencing or amplicon sequencing. In this case, sequencing only targets parts of the entire genome. This approach is often referred to as targeted sequencing.

A **"sequencing error profile"** or "error profile" refers to the list of parameters describing the rate at which artefactual base calls are introduced during the NGS workflow at each genomic position. The error profile accounts for different sources of artefacts, including PCR errors, sequencing errors and errors introduced at the read alignment step. The nature of the sequencing error profile depends on the nature of the statistical model using to generate synthetic alignment data. For example, if errors are modelled using a Beta-binomial distribution, the error profile may simply include two parameters defining the mean and the variance.

An **"adapter"** or **"adaptor"** refers to a short double-stranded or partially double-stranded DNA molecule of around 10 to 100 nucleotides (base pairs) which has been designed to be ligated to a DNA fragment. An adaptor may have blunt ends, sticky ends as a 3' or a 5' overhang, or a combination thereof. For example, to improve ligation efficiency, an adenine may be added to each of the 3' blunt ends of the fragmented DNA prior to adaptor ligation, and the adaptor may have a thymidine overhang on the 3' end to base-pair with the adenine added to the 3' end of the fragmented DNA. The adaptor may have a phosphorothioate bond before the terminal thymidine on the 3' end to prevent an exonuclease from trimming the thymidine, thus creating a blunt end when the end of the adaptor being ligated is double-stranded.

A **"partially double stranded adaptor"** refers to an adaptor including both a double-stranded region and a single stranded region. The double stranded region of the adaptor contains the ligation domain, whereas the single stranded region contains the primer sequences used for subsequent library amplification, barcoding and/or sequencing. The single stranded region can either be composed of two single stranded arms, a 5' arm and a 3' arm, as it is the case for so-called Y-shape adaptors, or the single stranded region of the partially double stranded adaptor can form a hairpin or a loop, as it is the case for so-called U-shape adaptors. The term partially double stranded adaptor refers thus both to Y-shape and U-shape adaptors or a combination thereof.

A group of **"PCR duplicates"** refers to a set of DNA products generated by PCR amplification from a single stranded DNA molecule belonging to a DNA-adaptor product derived from an original DNA fragment.

A **"molecular identifier"** or **"molecular tag"** or **"molecular barcode"** or **"molecular code"** refers to a feature of the DNA molecule that is used to identify PCR duplicates. Different types of molecular identifiers exist, including endogeneous UMIs (e.g., the mapping position of a DNA fragment) or exogenous UMIs (e.g., a random or pseudo-random sequence introduced in the DNA adaptor).

**"Aligning"** or **"alignment"** or **"aligner"** refers to mapping and aligning base-by-base, in a bioinformatics workflow, the sequencing reads to a reference genome sequence, depending on the application. For instance, in a targeted enrichment application where the sequencing reads are expected to map to a specific targeted genomic region in accordance with the hybrid capture probes used in the experimental amplification process, the alignment may be specifically searched relative to the corresponding sequence, defined by genomic coordinates such as the chromosome number, the start position and the end position in a reference genome. As known in bioinformatics practice, in some embodiments "alignment" methods as employed herein may also comprise certain pre-processing steps to facilitate the mapping of the sequencing reads and/or to remove irrelevant data from the reads, for instance by removing non-paired reads, and/or by trimming the adapter sequence at the end of the reads, and/or other read pre-processing filtering means.

**"Coverage"** refers to the number of sequencing reads that have been aligned to a genomic position or to a set of genomic positions. In general, a genomic region with a higher coverage is associated with a higher reliability in downstream genomic characterization, in particular when calling variants. In target enrichment workflows, only a small subset of regions of interest in the whole genome is sequenced and it may therefore be reasonable to increase the sequencing depth without incurring too significant data storage and processing overheads. In some genomic analysis applications not requiring a high resolution along the genome, for instance in detecting copy number alterations, low-pass (LP) coverage (1x-10x) or even ultra-low-pass (ULP) coverage (<1X - not all positions are sequenced) may be more efficient in terms of information technology infrastructure costs, but these workflows require more sophisticated bioinformatics methods and techniques to process the less reliable data output from the sequencer and aligner. Moreover, apart from the higher cost related to data storage and processing, the operational cost of an experimental NGS run, that is, loading a sequencer with samples for sequencing, also needs to be optimized by balancing the coverage depth and the number of samples which may be assayed in parallel in routine clinical workflows. Indeed, next generation sequencers are still limited in the total number of reads that they can produce in a single experiment (i.e. in a given run). The lower the coverage, the fewer reads per sample for the genomic analysis, and the higher the number of samples that can be multiplexed within a next generation sequencing run.

A **"molecular count"** or "count of molecules" refers to the number of distinct DNA molecules present in an original DNA sample for which at least one DNA product is observed in a DNA library or in alignment data.

**"Variant calling"** or **"variant caller"** or **"variant call"** refers to identifying, in the bioinformatics workflow, actual variants in the aligned reads. In bioinformatics data processing, a variant is uniquely identified by its position along a chromosome (*chr,pos*) and its difference relative to a reference genome at this position (*ref, alt*). Variants may include single nucleotide permutations (SNPs) or other single nucleotide variants (SNVs), insertions or deletions (INDELs), copy number variants (CNVs), as well as large rearrangements, substitutions, duplications, translocations, and others. Preferably variant calling is robust enough to sort out the real variants from the amplification and sequencing noise artefacts.

**"Mutation"** refers to a type of alteration in a nucleic acid sequence, such as a SNP, SNV or indel. A **"variant"** is a specific mutation occurring at a specific genomic position.

**"Variant allele fraction"** or **"Variant allele frequency"** or **"VAF"** is a measure of the fraction of DNA molecules in an original specimen carrying a variant. VAF can be measured in an NGS experiment by counting the number of sequencing reads that support a genomic variant divided by the overall coverage at that genomic position. Depending on the NGS assay, more sophisticated approaches may be used to measure VAF. For instance, when UMIs are used, PCR duplicates may be identified, and the VAF may be measured by counting the fraction of unique molecules supporting the variant, rather than the fraction of sequencing reads.

A **"statistical model"** or **"generative model"** is a computer-implemented mathematical model that assigns a probability to an instance of data generated by a stochastic process of interest. Statistical models can be divided in two subclasses: biophysical and machine learning models. A **"biophysical statistical model"** or a **"biophysical generative model"** is a statistical model derived from first principles incorporating insights into the biophysical process underlying the data generation process. Model parameters usually have a biophysical interpretation as well as physical units. In contrast, a **"machine learning statistical model"** or a **"machine learning generative model"** is a statistical model not entirely derived from first principles, but rather learnt from a training dataset using machine learning algorithms. In contrast to biophysical statistical models, machine learning statistical models can be derived without prior knowledge or understanding of the data generation process. The internal components and parameters of a machine learning model may not usually reflect biophysical processes and quantities.

Genomic analysis system

An exemplary genomic analysis system and workflow will now be described in further detail with reference to FIG.1. As will be apparent to those skilled in the art of DNA analysis, a genomic analysis workflow comprises preliminary experimental steps to be conducted in a laboratory (also known as the "wet lab") to produce DNA analysis data, such as raw sequencing reads in a next-generation sequencing workflow, as well as subsequent data processing steps to be conducted on the DNA analysis data to further identify information of interest to the end users, such as the detailed identification of DNA variants and related annotations, with a bioinformatics system (also known as the "dry lab"). Depending on the actual application, laboratory setup and bioinformatics platforms, various embodiments of a DNA analysis workflow are possible. FIG.1 describes an example of an NGS system comprising a wet lab system wherein DNA samples are first experimentally prepared with a DNA library preparation protocol 100 which may produce, adapt for sequencing and amplify DNA fragments to facilitate the processing by an NGS sequencer 110. In a next generation sequencing workflow, the resulting DNA analysis data may be produced as a data file of raw sequencing reads, for instance in the FASTQ format. The workflow may then further comprise a dry lab Genomic Data Analyzer system 120 which takes as input the raw sequencing reads for a pool of DNA samples prepared according to the proposed methods, and applies a series of data processing steps to characterize certain genomic features of the input samples. An exemplary Genomic Data Analyser system 120 is the Sophia Data Driven Medicine platform (Sophia DDM) as already used by more than 1000 hospitals worldwide in 2020 to automatically identify and characterize genomic variants and report them to the end user, but other systems may be used. Different detailed possible embodiments of data processing steps as may be applied by the Genomic Data Analyser system 120 for genomic variant analysis are described for instance in the international PCT patent application WO2017/220508, but other embodiments are also possible.

As illustrated on FIG.1, the Genomic Data Analyser 120 may process the sequencing data to produce a genomic data analysis report by employing and combining different data processing methods. The Genomic Data Analyser 120 may comprise a sequence alignment module 121, which compares the raw NGS sequencing data to a reference genome, for instance the human genome in medical applications, or an animal genome in veterinary applications. The sequence alignment module 121 may be configured to execute different alignment algorithms. Standard raw data alignment algorithms such as Bowtie2 or BWA that have been optimized for fast processing of numerous genomic data sequencing reads may be used, but other embodiments are also possible. The alignment results may be represented as one or several files in BAM or SAM format, as known to those skilled in the bioinformatics art, but other formats may also be used, such as compressed formats or formats optimized for order-preserving encryption and/or a combination thereof, depending on the genomic data analyser 120 requirements for storage optimization and/or genomic data privacy enforcement.

The resulting alignment data may be further filtered and analysed by a Variant Caller module 123 to retrieve variant information such as SNP and INDEL polymorphisms. The Variant Caller module 123 may be configured to execute different variant calling algorithms, either on the pre-processed consensus alignment data or directly on the alignment data for probabilistic variant callers, or on a set of pre-processed NGS data alignment features, as will be apparent to those skilled in the art of bioinformatics. Exemplary Variant Caller modules 123 which have been recently developed for somatic variant detection include Illumina Strelka2, GATK Mutect2, VarScan2, Shimmer, NeuSomatic, MuClone, MultiSNV, Needlestack, Qiagen smCounter or smCounter2, Sophia DDM probabilistic variant caller and others, for instance one of the 46 publicly available variant callers which may be applicable to single nucleotide variant detection as reviewed by Xu et al. in "A review of somatic single nucleotide variant calling algorithms for next-generation sequencing data", Computational and Structural Biotechnology Journal 16, pp. 15-24, Feb 2018.

In prior art bioinformatics NGS workflows, the variants detected by the Variant Caller 123 are typically reported as having a "positive" variant calling status. The corresponding detected variant information may then be output by the Genomic Data Analyser module 120 as a genomic variant report, for instance using the VCF (Variant Calling Format) file format, for further processing by the end user, for instance with a visualization tool, and/or by a further variant annotation processing module (not represented).

The Genomic Data Analyser 120 may be a computer system or part of a computer system including a central processing unit (CPU, "processor" or "computer processor" herein), memory such as RAM and storage units such as a hard disk, and communication interfaces to communicate with other computer systems through a communication network, for instance the internet or a local network. Examples of genomic data analyser computing systems, environments, and/or configurations include, but are not limited to, personal computer systems, server computer systems, thin clients, thick clients, handheld or laptop devices, multiprocessor systems, microprocessor-based systems, set top boxes, programmable consumer electronics, network PCs, minicomputer systems, mainframe computer systems, graphical processing units (GPU), and the like. In some embodiments, the computer system may comprise one or more computer servers, which are operational with numerous other general purpose or special purpose computing systems and may enable distributed computing, such as cloud computing, for instance in a genomic data farm. In some embodiments, the genomic data analyser 120 may be integrated into a massively parallel system. In some embodiments, the genomic data analyser 120 may be directly integrated into a next generation sequencing system.

The Genomic Data Analyser 120 computer system may be adapted in the general context of computer system-executable instructions, such as program modules, being executed by a computer system. Generally, program modules may include routines, programs, objects, components, logic, data structures, and so on that perform particular tasks or implement particular abstract data types. As is well known to those skilled in the art of computer programming, program modules may use native operating system and/or file system functions, standalone applications; browser or application plugins, applets, etc.; commercial or open source libraries and/or library tools as may be programmed in Python, Biopython, C/C++, or other programming languages; custom scripts, such as Perl or Bioperl scripts.

Instructions may be executed in distributed cloud computing environments where tasks are performed by remote processing devices that are linked through a communications network. In a distributed cloud-computing environment, program modules may be located in both local and remote computer system storage media including memory storage devices.

Genomic Data Analyzer

In a preferred embodiment, the Genomic Data Analyser system 120 may be adapted to produce an improved LOD-aware variant calling report from the analysis of alignment data in an NGS bioinformatics workflow. FIG.2 shows a possible system architecture for an LOD-aware variant caller 200 as an improvement of a conventional variant caller module 123. In a preferred embodiment, the LOD-aware variant caller 200 may be adapted to produce, with a statistical model 210, synthetic sequencing data and to automatically estimate from this synthetic sequencing data, with an LOD estimator module 230, a limit of detection for variant calling at a user-defined desired sensitivity. The proposed LOD-aware Variant Caller 200 may also classify, with a Variant Status Triage module 240, whether this variant is present (positive variant calling), absent at a high confidence (negative variant calling), or equivocal (possible false negative calling) for a user-defined minimum variant allele fraction (VAF) of interest.

In a preferred embodiment, the LOD-aware Variant Caller 200 comprises a Variant Caller module 123 adapted to operate with an NGS assay comprising library preparation 100, NGS sequencing 110 and NGS alignment 121 as known in the prior art. Depending on the choice of the NGS assay and workflow, different analytical factors may characterize it, such as its workflow error rate profile. As will be apparent to those skilled in the art of NGS genomic analysis, the NGS workflow error rate may be constant, or may vary with the variant positions and/or mutations.

In a possible embodiment, it may be possible to infer the molecular count present in the NGS data after NGS sequencing by combining information about the sample DNA amount, the Library Conversion Rate (LCR) profile as an additional analytical factor characterizing the NGS assay, and the read coverage.

A count of molecules in the DNA library may be first estimated by converting the sample DNA amount in genome equivalents (GE) or haplotype genome equivalents (hGE), and by accounting for the number of molecules present in the sample DNA that the NGS assay could not convert into a DNA library. For example, in a situation where the sample DNA amount is lOng and LCR=50%, then the count of molecules present in the DNA library may be estimated as 300[hGE/ng]*10[ng]*0.5 = 1500 molecules. Since library preparation is a stochastic process, the count of molecules in the DNA library may also be estimated using a stochastic variable. For example, it may be described by a Poisson distribution with an expected value set at 300*ng*LCR.

The molecular count present in the NGS data can then be further estimated by combining the molecular count present in the DNA library and the coverage depth measured from the alignment file. The LOD-aware Variant Caller 200 may measure, in the alignment data, the total coverage at the genomic position (*chr,pos*) of said variant. In a preferred embodiment, the total coverage value may be the sequencing read depth at the genomic position (*chr,pos*), but other embodiments are also possible, for instance only counting read depth of sufficient quality and other coverage measurement methods, as will be apparent to those skilled in the art of NGS bioinformatics. In cases where coverage depth is much larger compared to the DNA library molecular count, it is possible to assume that all molecules represented in the DNA library have been sequenced at least once. In this case, the count of molecules present in the DNA library provides a good estimation of the molecular count present in the NGS data. Otherwise, one has to consider that some molecules, while being present in the DNA library, may not be sequenced. In a possible embodiment, assuming that all molecule molecules represented in the DNA library are equally likely to be sequenced, the molecular count in the NGS data can be estimated as 300*ng*LCR*(1-exp(coverage/(300*ng*LCR)). In an alternate embodiment, since sequencing is a stochastic process, the molecular count in the NGS data may be described as a probability distribution with expected value equal to 300*ng*LCR*(1-exp(-coverage/(300*ng*LCR)) (e.g., Poisson distribution). Finally, in cases where the coverage depth is much smaller compared to the count of molecules in the DNA library, the number of unique molecules present in the NGS data tends to equal the coverage. In the latter scenario, the number of unique molecules present in the NGS data can thus be estimated simply by counting the number of reads in the NGS data.

In a possible embodiment, depending on its design, the NGS assay may enable the conversion of a patient sample DNA amount into a molecular amount depending on its Library Conversion Rate (LCR) profile as an additional, optional analytical factor characterizing the NGS assay. The LCR profile may be defined as a constant rate for all genomic positions or may vary with the genomic position. For example, the input molecular count may be calculated as 300*ng*LCR(*chr,pos*)*(1-exp(- coverage(*chr,pos*)/(300*ng*LCR(*chr,pos*)))), where 300*ng*LCR is the number of input DNA molecules which are converted into DNA product and successfully captured.

In an alternate embodiment, the NGS assay may employ an intrinsic molecular barcoding technology. Examples of molecular barcoding technologies include the use of Unique Molecular Identifiers (UMIs) which may be employed for consensus sequencing (*Xu et al., 2018, supra*). Examples of a Variant Caller 123 which exploits a molecular barcoding technology is described in "MAGERI: Computational pipeline for molecular-barcoded targeted resequencing", Shugay et al., PLoS Comput. Biol. 2017 May; 13(5) or in "smCounter2: an accurate low-frequency variant caller for targeted sequencing data with unique molecular identifiers", Xu et al., Bioinformatics, Vol,.35(8), April 2019. An alternate numerical coding technology to UMI barcoding is also described in co-pending patent application PCT/EP2020/076246. An alternate (less accurate) method to estimate a molecular count may comprise counting similar reads using the start and end positions in the alignment data in the absence of mapping positions. As will be apparent to those skilled in the art, any of the above molecular barcoding methods, when integrated in an NGS assay, enable to measure a molecular count for a variant directly from the alignment data.

Preferably, the LOD-aware Variant Caller 200 may be designed to operate with a diversity of NGS assay workflows, each NGS assay workflow being characterized by its analytical factors which can be defined once and stored as NGS assay pre-determined features 211 in a repository of the Genomic Data Analyzer system 120. The LOD-aware Variant Caller 200 may then retrieve the NGS assay pre-determined features 211 when analysing a variant depending on the NGS assay which has been applied to the patient DNA sample to be analysed.

In a preferred embodiment, the NGS assay analytical factors may comprise at least an NGS workflow error profile, and optionally an LCR profile, to characterize an NGS assay and possibly a sample type, but other embodiments are also possible. The analytical factors may be predetermined values stored in a memory in association with an NGS assay identifier to facilitate their retrieval by the LOD-aware variant caller, but other embodiments are also possible.

The LCR profile may be a constant value for all genomic positions, or a table of the library conversion rate value at each genomic position *(chr,pos)* or at sets of genomic positions. The LCR profile may also depend upon the DNA sample type.

The NGS assay error profile may be a constant value for all variants, a table of the error rate value at each variant position *(chr,pos)* or sets of positions, or a table of the error rate value for each variant (*chr,post,ref,alt*) or sets of variant types (*ref,alt*). The NGS assay error profile may also depend upon the DNA sample type.

In a preferred embodiment, as represented on FIG.2, the LOD-aware Variant Caller 200 may comprise a Variant Caller module 123 to detect whether a variant has a positive or non-positive status, a Generative Model module 210 to generate in silico synthetic alignment data for different VAFs, an LOD estimator module 230 to estimate a limit of detection for the Variant Caller operating on a variant of interest to the user at a user defined sensitivity and minimal VAF for said variant, and a Variant Status Triage module 240 to further classify the variant status as positive, negative or equivocal. In one embodiment, the LOD-aware variant calling results may be displayed to the end user on a graphical user interface. In another possible embodiment, the LOD-aware variant calling results may be produced as a text file, for instance as an extension of the VCF file format for further automated processing. Other embodiments are also possible.

LOD-aware variant calling workflow

FIG. 3 illustrates a possible workflow for the proposed LOD-aware variant caller 200 to estimate a limitation $LOD_{est}$ of detecting a nucleic acid sequence variant (*chr,pos,alt,ref*) in the alignment data (for instance a BAM file, but other embodiments are also possible) generated by a next-generation-sequencing assay from a patient sample, and for reporting the variant calling status as positive, negative or equivocal. Preferably, the variant may be an SNV or an INDEL, but other embodiments are also possible.

The proposed LOD-aware variant caller 200 may first acquire a BAM file for the patient sample, and determine, with a conventional variant calling module 123, the status of variant (*chr,pos,alt,ref*) in the alignment data.

If the variant call is positive, the LOD-aware variant caller 200 may then simply report its status as "positive" for instance in a VCF file, as in prior art variant calling systems. Yet if the variant call is non-positive, instead of either ignoring it or reporting it as negative (even though it is possibly a false negative) as prior art variant calling systems do, the LOD-aware variant caller 200 may apply the following steps:

- Obtain a molecular count measurement for the patient sample at the genomic position (*chr,pos*) of said variant;
- Obtain one or more analytical factors of the NGS assay used to process the patient sample, such as an NGS assay error profile and optionally an LCR profile;
- Produce, with a statistical model synthetic alignment data for one or more simulated VAFs as a function of the measured molecular count and of the analytical factors of the NGS assay;
- Estimate, from the synthetic alignment data, the detection sensitivity of said variant caller as a function of one or more of the simulated VAFs, for said assay, said DNA sample and said variant *(chr, pos, ref, alt)*.

In a possible embodiment, the LOD-aware variant caller generates, with a statistical model such as for instance a generative model, in silico alignment data as a function of the measured coverage, the measured molecular count and the analytical factors of the NGS assay. To produce in silico data, a number of simulated VAFs values may be selected in a range such as for example from 0.5%, 0.1%, 0.01% or less to 30%, 40%, 50% or more, depending on the application. For each of these simulated VAFs, a synthetic alignment data set may be produced as a BAM file, or alternately as a set of NGS data alignment features, as is suitable for processing by the conventional variant caller module 123. The conventional variant caller module 123 may then be called on each synthetic alignment data set to estimate the detection sensitivity specifically at the simulated VAF used to generate this data set. The LOD-aware variant caller may accordingly estimate a detection sensitivity function which is specific to the assay, the sample and the variant, by combining the sensitivity predictions of the different simulated datasets (each with a different simulated VAF).

In a further possible embodiment, the LOD-aware variant caller 200 may acquire a user-defined minimal variant allele fraction of interest (mVAF) for variant *(chr, pos, ref, alt)*, and classify the variant status as negative or equivocal as a function of the estimated detection sensitivity function and mVAF for this variant. To this end, the LOD-aware variant caller 200 may obtain a user-defined desired sensitivity for calling said variant as positive, estimate, from the estimated detection sensitivity function, the sensitivity for said mVAF, and classify the variant status as negative or equivocal as a function of said desired and estimated sensitivity, but other embodiments are also possible.

The LOD-aware variant caller 200 may accordingly report the variant calling status and/or the estimated sensitivity to the end user for the called variant *(chr,post,ref,alt)*, for instance in a file format extension to the VCF format for variant reporting, processing and/or storage, and/or using a dedicated graphical user interface to directly display the results to the end user.

In another possible further embodiment, the LOD-aware variant caller 200 may obtain a user-defined target sensitivity for variant *(chr, pos, ref, alt)* and estimate, from the estimated detection sensitivity function, a limit of detection $LOD_{est}$ value for this variant as the lowest VAF detectable with a sensitivity larger or equal to the user-defined sensitivity. The LOD-aware variant caller 200 may also obtain both a user-defined minimal variant allele fraction of interest (mVAF) and a user-defined desired sensitivity for calling variant *(chr, pos, ref, alt)* as positive, estimate from the estimated detection sensitivity function a limit of detection $LOD_{est}$ value as the lowest simulated VAF detectable with a sensitivity larger or equal to the user-defined sensitivity and classify the variant status as negative if the mVAF for said variant is larger or equal to $LOD_{est}$, or as equivocal otherwise.

The LOD-aware variant caller 200 may accordingly report the estimated $LOD_{est}$ value and/or the variant calling status result to the end user for the called variant *(chr,post,ref,alt)*, for instance in a file format extension to the VCF format for variant reporting, processing and/or storage, and/or using a dedicated graphical user interface to directly display the results to the end user.

Experimental results

An exemplary embodiment will now be described, based on a carefully controlled experiment to define the factors governing NGS assay limitations, using circulating tumour DNA profiling as a paradigm. This uncovered a complex interplay between technical factors and genomic context, enabling the development of the in-silico method that predicts the limit of detection (LOD, the lowest detectable variant fraction) for every position interrogated by an assay. This method may be integrated into an LOD-aware variant calling framework 200 that uses LOD predictions to flag potential false negatives. Applying this to 580 clinical samples predicted all but one false negative call, resulting in an inter-assay concordance of 99%. The proposed approach improves the reliability, reproducibility and transparency of genetic testing, with widespread benefits for clinical trials, technology development and patient care. The proposed approach includes an LOD-aware variant calling framework 200 that predicts and reports LODs alongside NGS results. This approach may be developed to operate with a diversity of NGS assays. For the carefully controlled experiment, ctDNA assays were used, as these represent a challenging test case that can benefit greatly from reliability improvements.

A complex interplay between technical factors and genomic position defines LOD, with contributing factors including input DNA amount, library conversion rate, sequencing coverage and PCR/sequencing error rate. Variation in these factors leads to a remarkable variability in LOD between samples, NGS technologies and genomic positions. Drawing upon these insights, an in-silico approach may be developed to accurately predict the LOD for every genomic position interrogated by an NGS assay. It may then be integrated into an LOD-aware variant calling framework 200 that introduces a third label for variant calls based on technical limitations. Specifically, negative calls may be triaged into high confidence results versus potential false negatives (now labelled "equivocal").

## Example experiments

**A1** (NGS assay A1, amplicon-based); **A2** (NGS assay A2, capture-based); **A3** (NGS assay A3, capture-based with molecular barcoding); **BEAMing** (Beads, Emulsion, Amplification and Magnetics); **cfDNA** (cell-free DNA); **ctDNA** (circulating tumour DNA); **dPCR** (digital PCR); **ddPCR** (droplet digital PCR); *EGFR* (gene coding epidermal growth factor receptor); **gDNA** (genomic DNA); *KRAS* (gene coding K-Ras protein); **LCR** (library conversion rate); **LOD** (limit of detection); **NGS** (next-generation sequencing); **NSCLC** (non-small cell lung cancer); **rsctDNA** (reference standard circulating tumour DNA); **SNVs** (single nucleotide variants); **VAFs** (variant allele frequencies or fractions).

### Example 1: LOD is determined at multiple scales

The limit of detection (LOD) of next-generation sequencing (NGS) assays was defined as described below.

### *Materials and methods*

*Clinical cfDNA samples:* Samples were collected within the framework of the CIRCAN ("CIRculating CANcer") study, which is a routine program established to comprehensively evaluate tumour biomarkers in cell-free DNA (cfDNA) from non-small cell lung cancer (NSCLC) patients at the Lyon University Hospital (Hospices Civils de Lyon, HCL). The main inclusion criteria were (i) that patients were histologically or cytologically diagnosed as having metastatic NSCLC, and (ii) that these patients had undergone molecular testing for epidermal growth factor receptor (EGFR) mutations in tumour biopsies (routinely performed in France) *(Garcia et al., 2017, Oncotarget, Vol 8, No 50; Garcia et al., 2018, Oncotarget, Vol 9, No 30)*. Patient inclusion was initially limited to patients treated in the HCL and in countryside hospitals in the Rhone-Alpes region.

Plasma was prepared from 10-25 mL of blood collected in $K_2$ EDTA (ethylenediaminetetraacetic acid) tubes (BD, 367525, 18 mg). All blood samples were delivered to the laboratory within 24 hours of collection. Detailed pre-analytical considerations have previously been published *(Garcia et al., 2017, supra)*. CfDNA was extracted from 4 mL or 8 mL of plasma using the QIAamp Circulating Nucleic Acid Kit (Qiagen, Cat No 55114, Valencia, CA, USA), with a Qiagen vacuum manifold following the manufacturer's instructions. CfDNA was then eluted in a final volume of 60 μL of elution buffer (AVE; Qiagen, part of Cat No 55114) depending on the volume of plasma used for the extraction (3 mL or 8 mL).

*Preparation of reference ctDNA: (1) rsctDNA (reference standard circulating tumour DNA) and (2) sonicated gDNA (genomic DNA)*

*Enzymatic shearing (rsctDNA):* Nucleosomal DNA was generated from seven different human cell lines (GM07048, GM14638, GM14097, GM14093, GM12707, GM12815 and GM11993, Coriell Institute, with the EZ Nucleosomal DNA Prep (Zymo Research, Cat. No. D5220) using the Atlantis dsDNase treatment (Zymo Research, part of Cat. No. D5220) according to the manufacturer's instructions with minor modifications, as follows. After collection, cells were stored at -80°C. For each cell line, a pellet of $10^6$ cells was thawed on ice, resuspended in 1 ml ice-cold lysis buffer (10 mM Tris-HCl pH 7.5, 10 mM NaCl, 3 mM $MgCl_2$, 0.5 % NP-40, 0.5 mM Spermidine) and incubated for 5 min on ice. The resulting nuclei were then washed once with 200 μl ice-cold Atlantis digestion buffer, then resuspended in 100 μl Atlantis digestion buffer with 0.25 U Atlantis dsDNAse (double-stranded DNA-specific endonuclease) and incubated for 1 h at 42°C. To stop the digestion, 18 μl of Stop solution (75 mM EDTA, 1.5 % SDS, 0.7 M NaCl) was added. Samples were then treated with RNaseA (ribonuclease) (0.3 mg/μl) for 15 min at 42 °C, then with proteinase K (1 mg/ml) for 30 min at 37 °C. Following enzymatic treatments, DNA was column purified with the Zymo Genomic DNA Clean & Concentrator kit (Zymo Research, Cat. No. D4065). To remove large and small DNA fragments, samples were subjected to a dual size selection using AMPure XP Beads (Beckman Coulter) with bead:DNA ratios of 0.7x and 1.5x. After fluorometric quantification, the nucleosomal DNAs of GM07048, GM14638, GM14097, GM14093, GM12707 and GM12815 were spiked into the nucleosomal DNA of GM11993, generating DNA mixes (D1, D2 and D3; dilution 1, 2 and 3 respectively) with variants at the indicated variant fractions (VAF):

| Variant | Gene | Expected VAF D1 | Expected VAF D2 | Expected VAF D3 |
|---|---|---|---|---|
| 1:115256414_C:T | NRAS | 0.50% | 0.25% | 0.10% |
| 2:29445458_G:T | ALK | 0.50% | 0.25% | 0.10% |
| 2:198266862_A:T | SF3B1 | 0.50% | 0.25% | 0.10% |
| 2:209113192_G:A | IDH1 | 0.50% | 0.25% | 0.10% |
| 2:212566786_G:T | ERBB4 | 0.50% | 0.25% | 0.10% |
| 3:138665029_G:C | FOXL2 | 0.50% | 0.25% | 0.10% |
| 3:138665064_G:A | FOXL2 | 0.50% | 0.25% | 0.10% |
| 4:1803704_T:C | FGFR3 | 1.00% | 0.50% | 0.20% |
| 4:55152040_C:T | PDGFRA | 1.50% | 0.75% | 0.30% |
| 4:55593464_A:C | KIT | 0.50% | 0.25% | 0.10% |
| 4:55602765_G:C | KIT | 0.50% | 0.25% | 0.10% |
| 7:55249063_G:A | EGFR | 1.00% | 0.50% | 0.20% |
| 7:55259450_C:T | EGFR | 0.50% | 0.25% | 0.10% |
| 7:116339282_G:A | MET | 0.50% | 0.25% | 0.10% |
| 7:116339672_C:T | MET | 0.50% | 0.25% | 0.10% |
| 7:116411923_C:T | MET | 0.50% | 0.25% | 0.10% |
| 9:21970916_C:T | CDKN2A | 0.50% | 0.25% | 0.10% |
| 10:43610119_G:A | RET | 0.50% | 0.25% | 0.10% |
| 10:43613843_G:T | RET | 4.00% | 2.00% | 0.80% |
| 10:43615633_C:G | RET | 0.50% | 0.25% | 0.10% |
| 11:534332_G:A | HRAS | 0.50% | 0.25% | 0.10% |
| 12:112888239_C:T | PTPN11 | 0.50% | 0.25% | 0.10% |
| 17:7576841_A:G | TP53 | 0.50% | 0.25% | 0.10% |
| 17:7579579_C:T | TP53 | 0.50% | 0.25% | 0.10% |

*Mechanical shearing (sonicated gDNA):* Sonicated genomic DNA was prepared using an E220 Evolution sonicator (Covaris) according to the manufacturer's instructions, to obtain an average size of 150 bp. Briefly, for each sample, 1 µg of DNA in 55 µl of TE buffer was added to a Rack E220e 8 microTUBE Strip V2 and sheared using the following parameters: Peak Incident Power (W): 75, Duty Factor: 15 %, Cycles per Burst: 500, Treatment Time: 360 s.

*Fragment size analysis of reference ctDNA and ctDNA from clinical samples:* The cfDNA size from clinical samples were assessed using the Agilent 2100 BioAnalyser (Agilent Technologies, Santa Clara, CA, USA) and the DNA High Sensitivity kit (Agilent Technologies, Santa Clara, CA, USA, 5067-4626 & 5067-4627). Two size-standardized internal controls (of 35 bp and 10,380 bp) and a DNA ladder (15 peaks) were used in each bioanalyser runs. The profile of fragment sizes was generated using the 2100 Expert Software (Agilent Technologies, Santa Clara, CA, USA).

*NGS library preparation and sequencing*

*NGS assay A1.* Targeted libraries were created using a multiple targeted amplicon kit (Accel-Amplicon 56G Oncology Panel v2, AL-56248, Swift Biosciences) according to the manufacturer's instructions.

The kit enables the detection of mutations present in a set of clinically relevant genes implicated in cancers. cfDNA samples with inputs varying from 2.2 to 37.7 ng were subjected to an initial multiplex PCR using the panel specific set of primers and 25 cycles of amplification. PCR products were purified using AMPure beads (Beckman Coulter). Finally, dual-index adapters, provided as part of the kit, were ligated to the purified PCR products prior to a final AMPure bead purification step (Beckman Coulter). *NGS assay A2:* Targeted libraries were created using capture-based enrichment technology. First, 10-50 ng of input cfDNA was end-repaired and A-tailed, followed by ligation to Illumina dual-indexed adapters. Ligation products were purified using AMPure beads (Beckman Coulter) and further amplified by PCR for 10 to 14 cycles depending on the amount of input DNA. Amplified libraries were cleaned-up using AMPure beads (Beckman Coulter) and then libraries pooled to give a total of 1.8 $\mu$g. The pools were mixed with human Cot-1 DNA (Life Technologies) and xGen Universal Blockers-TS Mix oligos (Integrated DNA Technologies) and lyophilized. Pellets were resuspended in a hybridization mixture, denatured for 10 min at 95°C and incubated for 4-16 h at 65°C in the presence of biotinylated probes (xGEN Lockdown IDT®). The probe panel spanned 170 Kb and covers a set of clinically relevant genes implicated in cancer that partially overlaps with the panel of assay A1. Probe-hybridized library fragments were captured with Dynabeads M270 Streptavidin (Invitrogen) and then washed. The captured libraries were amplified by PCR for 14 cycles and cleaned-up using AMPure beads (Beckman Coulter).

*NGS assay A3.* Targeted libraries were created using a capture-based enrichment technology including molecular barcodes. First, 10-50 ng of input cfDNA was end-repaired and A-tailed, followed by ligation to short y-shaped adapters with a double-stranded molecular barcode of 4-5 bp. The ligation products were purified with AMPure beads (Beckman Coulter) and then amplified for 10 to 14 cycles (depending on the amount of input DNA) using Illumina-compatible primers with dual-indices. Amplified libraries were cleaned-up with AMPure beads (Beckman Coulter). Targeted enrichment of pooled libraries was performed as for NGS assay A2 but using one more PCR cycle (i.e. 15 instead of 14). This is because the probe panel for assay A3 has a smaller footprint (56 Kb) than that of assay A2 (note that panel A3 also targets genes clinically relevant for cancer). PCR products were finally purified using AMPure beads (Beckman Coulter).

*Sequencing*

Prior to sequencing, libraries were quantified by qPCR and normalised to 0.5 nM for NGS assay A1 and 4 nM for NGS assays A2 and A3. All libraries were sequenced using a NextSeq 500 sequencer (Illumina) with either a Mid-Output or High-Output kit as specified by the manufacturer. Paired-end sequencing reads of 150 bases were generated (2x150 cycles) together with two index reads of 8 bases. Sonicated gDNA and rsctDNA libraries were loaded on the sequencer such that coverage was proportional to the input material and comparable between the different size panels (A1: 2800x/ng; A2: 2200x/ng, and 1900x/ng, corresponding to at least 6 reads per molecule on average). This guarantees that most molecules converted into library were sequenced at least once. Clinical samples were sequenced as per the assay manufacturers' instructions.

*Data processing and standard variant calling*

*NGS data demultiplexing, preprocessing and alignment:* Demultiplexing (and molecular barcode trimming for assay A3) was performed on base-call files (BCL) using bcl2fastq2 and the resulting fastq files aligned against the human genome reference Hg19 (GRCh37.p5) using bwa *(Li & Durbin, 2009, Bioinformatics 25, 1754-1760).* For each genomic position a pileup was performed by counting reads with a Phred quality score >15 supporting the reference or alternative allele. For assay A3, groups of PCR duplicates were collapsed to consensus sequences prior to the pileup step *(Schmitt et al., 2012, PNAS 109, 14508 LP - 14513).* First, pairs of reads were grouped into DNA molecules using their mapping positions and molecular barcode sequences. Molecules for which only one of the two strands were sequenced were discarded. For each remaining molecule a base call consensus was obtained for each strand. 70% base call consistency within a strand to form a consensus was required. Finally, a consensus was obtained for the two strands, producing a single consensus sequence for each DNA molecule. Alternative bases needed to be present in both single strand consensuses to be retained in the final consensus.

*Standard variant calling:* First, a background noise model was generated for each assay, genomic position and mutation (three SNVs, insertion and deletion) using NGS data from characterised samples. For assay A1, 136 Tru-Q samples (Tru-Q 0, 5, 6 and 7, Horizon Discovery) routinely included as controls in clinical runs were used. For assay A2, 181 germline blood samples included in clinical runs were used. For assay A3, 10 negative samples included as controls with the rsctDNA samples were used (these are samples produced entirely from the background cell line). For a given depth and mutation (three single nucleotide variants (SNVs), insertion or deletion) the background noise model is a fitted distribution of the number of alternative reads produced by technical noise, such as sequencing errors. For assays A1 and A2 a beta-binomial distribution to the measured background noise profile was fitted, which was able to represent the observed overdispersion (Ramu et al., 2013, Nat. Methods 10, 985-987). For assay A3 technical noise was lower as PCR duplicates were collapsed to consensus sequences. This permitted fitting of a simpler Binomial model. Using flat priors, the beta-binomial was fitted to the data using maximum likelihood estimation, while the mean a posteriori approach was used to obtain the rate for the binomial model. For model fitting, confirmed variants were excluded (as these would skew the background noise estimation and were not noise). Also excluded were positions with a VAF larger than 25%, as these were likely to represent true variants. In cases where a position had a variant in all samples and thus no data were available for the fit, the parameters were set to the average of the background noise of neighbouring positions. To call variants, the probability that the observed number of reads supporting the alternative allele $N_{ALT}$ was generated by the background noise model, was calculated i.e.,

$$P\left(N_{ALT}^{Noise} > N_{ALT}\middle|N, \theta\right)$$

$$(\text{Eq. } 1)$$

where $\theta$ represents the parameters of the fitted beta-binomial or binomial for a given genomic position and variant type (three SNVs, insertion or deletion). The negative logarithm of this probability was taken, and variants were called based on whether this score was above or below a defined threshold. The threshold value $\alpha$ was chosen to ensure a similar false positive rate, measured across all reference positions for the reference standard samples, and was 50, 50 and 7 for assays A1, A2 and A3 respectively.

*Collision rate calculation*

As used herein, the term "collision" means the event in which two, or more than two, different DNA molecules were fragmented the exact same way, and thus cannot be distinguished by their mapping position alone. The collision rate is the rate at which such events occur, and a high collision rate indicates that molecular barcodes are necessary to accurately quantify molecular counts. More precisely, the number of mapping positions at which more than one molecule aligned was counted and divided by the total number of mapping positions occupied in the data.

*Theoretical LOD for a given input amount*

To evaluate the impact of input material on sensitivity a minimal model was developed that assumes that coverage is in excess (i.e. all molecules incorporated into the library will be sequenced) and neglects sequencing noise. Thus, the ability to detect variants is solely limited by the number of molecules in the input material supporting the variant, $M_{ALT}$, and the library conversion rate, LCR. Here it was assumed that $M_{ALT}$ follows a Poisson distribution with mean $300 \times Input \times VAF \times LCR$, and that the probability of detecting a variant with a given VAF and input material was given by:

$$P(M_{ALT} \geq k|VAF, Input, LCR)$$

$$(\text{Eq. } 2)$$

where k is the minimum number of molecules required to make a call. It was assumed that at least two molecules were required to make call. An LCR of 100% for the maximum theoretical sensitivity was used. These data are presented in **Figure 6D**. As this figure aggregates different VAFs, the average sensitivity over the VAFs present in the analysed samples was computed. This model was also used as a basis to infer the LCR for assay A3 (Library conversion rate estimation, NGS assay A3 below) (
**Figure 6F-H).**

*Library conversion rate estimation*

The library conversion rate was defined as the fraction of DNA molecules present in the sequencing data divided by the number of DNA molecules present in the sample, given by the number of haploid genomes per ng (300) multiplied by the mass of input material (in ng). For this calculation, the number of molecules in the sample needs to be determined.
NGS assay A2 and A3: For assay A3 the number of molecules can readily be measured by identifying PCR duplicates using the fragments' mapping positions and molecular barcodes. The LCR was computed per targeted region by retaining fragments that overlapped the centre of the region, and an average LCR computed by averaging over genomic regions. The same method was applied for assay A2, but since this assay was not equipped with molecular barcodes the resulting LCR was likely to be underestimated, as two molecules mapping as the same position (a collision) was counted as one. However, replicates were used to estimate the collision rate and correct for this bias. The frequency at which molecules map at the same position can be estimated by counting the number of mapping positions occupied in both replicates and dividing by the total number of occupied mapping positions. It was found that for 5 ng of input material the collision rate was 6%. The LCR was thus corrected by inflating the observed number of molecules by the collision rate.

NGS Assay A1: Since assay A1 was not equipped with molecular barcodes and the mapping positions were the same for all fragments in an amplicon, it was not possible to rely on molecular counts to estimate the LCR for assay A1. Therefore, the observed variant calls for rsctDNA samples exploited instead. The model for the number of molecules supporting the variant, $M_{ALT}$, defined as above (in Theoretical LOD for a given input amount) was used, and it was assumed that at least two molecules were required to make a call, as it gave good agreement with the data **(Figure 6A).** For a given variant *i* with a VAF $VAF_i$ and input amount $Input_i$ the probability of the call (i.e. called or missed) was given by:

$$P(Call_i|LCR) = \begin{cases} P(M_{ALT} > k|VAF_i, Input_i, LCR) \text{ if the variant was called} \\ P(M_{ALT} \le k|VAF_i, Input_i, LCR) \text{ if the variant was missed} \end{cases}$$

$$(\text{Eq. } 3)$$

The likelihood of observing the calls, as a function of LCR, was then given by:

$$P(Calls|LCR) = \prod_{i=1}^{N} P(Call_i|LCR) \qquad (\text{Eq. } 4)$$

Assuming a flat prior for the LCR, the likelihood was then numerically integrated to obtain the posterior distribution $P(LCR|Calls)$, which is plotted in **Figure 6B.**

*Variant concordance analysis:* To measure the concordance between the three NGS assays, or between NGS assays and PCR-based methods, the fraction of confirmed variants that had the same call status (positive or negative) in all assays was computed. For LOD-aware variant-calling all variants that had at least two non-equivocal calls were selected, and concordance computed amongst these. For example, the situation in which two assays made a positive call and the third one made an equivocal call was counted as concordant, while the situation in which only one of the three assays made a non-equivocal call was excluded from the concordance analysis.

## *Results*

To accurately predict the LOD of NGS assays required a detailed understanding of how LOD is defined. Therefore, there was a need for an experimental system where technical factors such as input amount, assay biases and sequencing errors could be studied individually and in combination. This was impractical using clinical samples, due to their heterogeneity and scarcity, so an artificial reference material was sought. A range of these were available, and as it was unclear which was most commutable with clinical samples *(*Geeurickx & Hendrix, 2020, Mol. Aspects Med. 72, 100828) two possibilities were tested. The first, "reference standard ctDNA" (rsctDNA), comprises DNA purified from endonuclease-digested chromatin and mimics circulating tumour DNA (ctDNA) production *in vivo (*Snyder et al., 2016, Cell 164, 57-68*).* Commutability of sonicated gDNA and rsctDNA, from endonuclease-digested chromatin with clinical ctDNA, was assessed using a capture-based NGS assay with molecular barcodes (assay A3; **Figure 4**). Collision rate **(Figure 4A),** fragment size distribution **(Figure 4B),** distribution of error rates at genomic positions (n=6400) within the target region shared by all assays **(Figure 4C),** and the average number of unique molecules covering each interrogated genomic position **(Figure 4D)** were assessed.

Comparing NGS libraries from rsctDNA and clinical ctDNA revealed similar molecular features *(*Mouliere et al., 2018, Sci. Transl. Med. 10, eaat4921*),* whereas the second reference material, sonicated genomic DNA (gDNA), produced a less complex library **(Figure 4A-D)**. It was concluded that rsctDNA is the more clinically analogous reference.

Next, an NGS experiment using rsctDNA to examine determinants of LOD was designed, as shown in **Figure 5A.** Three dilutions of rsctDNA (i.e., D1, D2 and D3), each containing 13 variants at various VAFs (i.e., D1 0.5-4%; D2 0.25-2%; D3 0.1-0.8%), were prepared by mixing DNA isolated from endonuclease-digested chromatin extracts (from seven cell lines). Three rsctDNA amounts (i.e., 5ng, 10 ng and 25ng), three dilutions (i.e., D1, D2 and D3, each containing 13 single nucleotide variants, SNVs, with a clinically relevant VAF range of 0.1-4% *(*Matsumoto et al., 2020, Lung Cancer 139, 80-88*;* Jiang et al., 2019, Mol Med Rep 20, 593-603*)),* and three widely used NGS technologies (amplicon-based (assay A1), capture-based (assay A2) and capture-based with molecular barcoding (assay A3)) were included. NGS data were used for variant calling, and the results used to assess concordance between NGS assays and determine factors governing sensitivity/LOD. It was first evaluated whether this setup yielded a similar analytical performance to that observed in clinical studies, by examining variant calls for the known SNVs. This revealed abundant false negatives, particularly for low VAFs **(Figure 5B),** as seen in clinical studies. Consequently, the overall LOD was 0.2-0.7% and concordance between assays low (44% for VAFs ≤1%) **(Figure 6A-B).** Notably, the sensitivity (proportion of confirmed SNVs detected) of assay A3 was lower when this experiment was repeated using sonicated gDNA **(Figure 6C),** confirming that rsctDNA is the better reference choice. Overall, the low inter-assay concordance resembles that seen in clinical studies (22-55%) *(Stetson et al., 2019, supra; Kuderer et al., 2017, supra;* Jovelet et al., 2016, Clin. Cancer Res. 22, 2960 LP - 2968*),* confirming that this model was realistic.

To determine which factors influence LOD, variant calls (true positive or false negative) were plotted for the 13 SNVs arranged by rsctDNA dilution, NGS assay, and input amount **(Figure 5C).** The manner in which sensitivity, i.e. the proportion of true positives, varies between these conditions was then examined. As sensitivity is inversely related to LOD, this reveals factors contributing to LOD. As expected, sensitivity was lowest for low input amounts or VAFs **(Figure 5C, Figure 6A** and **D**

). There were also clear differences between NGS technologies, which could be due to variation in the extent to which PCR or sequencing noise obscure faint signals. Indeed, the most sensitive assay (A3) had the lowest error rate **(Figure 6D-E).** Nonetheless, assay A3 did not achieve the maximum sensitivity possible given the amount of input material **(Figure 4D),** implying that additional factors also limit sensitivity. One candidate was library conversion rate (LCR), the efficiency with which DNA is incorporated into an NGS library. Therefore, the LCR was computed for each assay, either by counting detected molecules (distinct fragments in the NGS library) or, as this approach was not possible for assay A1, using a model that assumes LCR is limiting **(Figure 6F-H).** Overall, LCR correlated with sensitivity. It was therefore concluded that LCR, error rate, and input amount may all govern sensitivity and thus LOD.

Importantly, for a given assay, input amount, and rsctDNA dilution, variants with the same VAF were detected with markedly different sensitivities **(Figure 5C,** light grey (low VAF) variants). This implies that LOD depends strongly on genomic context. Consistent with this, determinants of LOD (error rate, sequencing coverage and LCR) fluctuate between genomic positions **(Figure 5D).** It was concluded that to accurately predict LOD, the complex interplay between technical factors acting at assay, sample and nucleotides levels must be taken into account.

**Example 2: Adaptive LOD predictions resolve NGS assay discordance**

A variant calling framework that identifies and reports potential incorrect calls was developed as follows.

***Materials and methods***

*Materials and methods as in Example 1, in addition to the following:*

<u>*LOD-aware variant calling*</u>

*Generative model:* To predict LOD and sensitivity more generally than described in Example 1 (i.e. for different amounts of input material or higher VAFs), and to take account of background noise, a generative model to simulate the entire NGS workflow was developed, with simulated data then interpreted by a variant caller.

*Variant caller:* A variant caller can be seen as a function *VC* mapping a set of observed features *F* (like coverage, number of reads supporting the variants, etc.) to a score that is used to take a decision, given a threshold $\alpha$, on the presence of a variant: VC(*F*) > $\alpha$. To obtain the probability distribution of the features *F* given a predefined set of parameters (Input material, VAF, etc.), i.e. P(*F*|$\theta$), a generative model was used (*Generative model for NGS assay A1, A2 and A3,* below). The predicted sensitivity could then be computed by integrating the positive calls over the set of features:

$$\text{Predicted sensitivity} = \int_{F} (VC(F) > \alpha) P(F|\theta)$$

$$(Eq. 5)$$

Depending on the complexity of the feature set and of the generative model this integral could either be solved analytically, integrated numerically, or approximated using sampling techniques *(*Davis & Rabinowitz, Methods of Numerical Integration, Dover Publications, 2007*).* Here, since our feature set was small and our generative model was relatively simple, numerical integration was used to solve the integral. Importantly, since the predicted sensitivity is a function of the *VAF* (one of the features within the feature set *F*), one could solve the equation above for the *VAF* corresponding to a desired predicted sensitivity (i.e. the LOD).

*Generative model for NGS assay A1 and A2:* The generative model for assays A1 and A2 aimed at producing the distribution of the number of reads supporting the variant, $N_{ALT}$, given the total number of reads $N$ (coverage depth, known from the NGS data), the input material, the LCR, a VAF and a background noise model. It was reasoned that at low variant fraction, the fluctuations in the number of alternative molecules and alternative reads would play a larger role than fluctuations in the total number of molecules and reads. Therefore, integration was performed over the distributions for the former and averages used for the latter. Thus, the mean total number of molecules $M$ was on average $300 \times Input \times LCR,$ and the number of molecules supporting the variant, $M_{ALT}$, followed a Binomial distribution with mean $300 \times Input \times LCR \times VAF$. It was assumed that molecules were amplified homogeneously into pools of PCR duplicates and reads were sequenced randomly from these pools. Thus, the mean number of sequenced reads per molecule was given by $\lambda = N/M$, and the total number of reads supporting the variant $N_{ALT}$ follows a Poisson distribution with mean $\lambda \times M_{ALT}$. The contribution of the background noise was added to this, with the number of artefactual reads supporting the variant following a beta-binomial distribution with parameters estimated as explained above (*Standard variant calling*). The predicted sensitivity was finally computed as:

$$\sum_{M_{ALT}} \sum_{N_{ALT}} \sum_{N_{ALT}^{Noise}} \left(VC\left(N_{ALT} + N_{ALT}^{Noise}, N\right) > \alpha\right) P\left(N_{ALT}^{Noise}\right) P(N_{ALT} \mid M_{ALT}) P(M_{ALT})$$

(Eq. 6)

Instead of integrating on the whole support of the distributions, the sum was restricted to the $10^{-5}$ and $1\text{-}10^{-5}$ quantiles of the distributions, which was sufficient to produce an accurate estimation of predicted sensitivity.

*Generative* model for NGS assay A3: Since data produced by assay A3 were deduplicated to form consensuses, each resulting read-pair corresponds to a single DNA molecule, so the generative model for A3 only needed to produce the distribution of the number of alternative molecules supporting the variant $M_{ALT}$ given the total number of molecules $M$ (coverage of duplexes, known from the NGS data), a *VAF* and a background noise model. As in the previous section, the number of alternative molecules followed a Binomial distribution with mean $M \times VAF,$ and the contribution of the background noise (Binomial distribution) was added to obtain the total number of alternative molecules provided to the variant caller. Thus, the predicted sensitivity was:

$$\sum_{M_{ALT}} \sum_{N_{ALT}^{Noise}} \left(VC\left(M_{ALT} + M_{ALT}^{Noise}, M\right) > \alpha\right) P\left(M_{ALT}^{Noise}\right) P(M_{ALT})$$

Eq. (7)

### *Results*

Having identified factors that may lead to false negatives and discordance between NGS assays, these factors were used to develop a variant calling framework that identifies and reports potential incorrect calls. In a standard workflow (**Figure 7A**, top), sequencing data are interpreted by a variant caller that outputs positive calls but does not report positions where no variant was detected. Our "LOD-aware" framework has built on this by using an in silico generative model (**Figure 7A**, encapsulated in thick dashed lines) to predict LODs for every interrogated position, which ultimately enabled positions to be triaged into positive, negative and equivocal classes (**Figure 7A**, right). The inputs to this model were LCR and PCR/sequencing error rates, calculated when setting up an assay, and input amount and sequencing coverage, measured for each sample. Sequencing coverage and error rates were defined for each genomic position. The model simulated an NGS experiment *in silico,* following biophysical rules and incorporating stochastic sampling effects to produce synthetic sequencing data. Specifically, for each potential variant in a sample, DNA fragments were simulated for various VAFs, converted into a library and amplified by PCR. Sequencing reads were then sampled to the appropriate depth and errors injected. A variant caller processed the *in-silico* data to generate a curve of sensitivity vs VAF, from which the predicted LOD was read off (**Figure 7A,** dark grey block arrow marked 'LOD prediction'; LOD is defined as the lowest VAF detectable with the required sensitivity). For assay A3, the model was adjusted to reflect the inclusion of molecular barcodes (**Figure 8A**).

The output from this model could then be used for LOD-aware variant calling, which takes assay limitations directly into account. Specifically, whereas standard variant callers omit positions where no variant was called, this strategy both reports these positions and assigns one of two possible labels: "equivocal", where a high (insufficient) predicted LOD flags a potential false negative; or "negative", where a low predicted LOD indicates we would have detected a variant if present **(Figure 7A,** right). For positive calls, this approach can flag "near misses", where a high LOD indicates this variant will not be detected in future, similar samples.

Having established an LOD-aware variant calling framework, its accuracy was checked using data from the rsctDNA experiment. Specifically, the framework was used to predict sensitivities for confirmed variants, grouped variants by these predictions, then the experimentally measured sensitivities plotted for each group **(Figure 8B** and **C).** The close correlation between predicted and observed sensitivities confirmed the accuracy of the above predictions and implied that the factors that were identified **(Figure 5)** and included in the model sufficiently explain LOD. Next, it was tested whether this LOD-aware strategy predicts which variants were missed during variant calling for the rsctDNA data. It was assumed that variants with predicted sensitivities >50% would be detected, and remaining variants missed. This strategy correctly predicted variant calling results for 78%, 88% and 90% of the confirmed variants in assays A1, A2 and A3. It was concluded that this framework accurately predicts LOD and sensitivity and identifies when a variant caller will miss a variant.

This approach has several practical applications. First, predicted sensitivities can be used to benchmark or compare NGS assays. Second, by reporting LODs alongside variant calls and flagging potential false negatives as "equivocal", this approach can help clinicians decide when retesting is required. Here, clinical insight is used to define a minimal VAF of interest, such as the lowest VAF proven to predict treatment response. Equivocal calls are then denoted as positions where LOD was insufficient to guarantee detection of a variant down to this threshold. To demonstrate this using the above rsctDNA dataset, the known VAFs (defined during rsctDNA preparation) were used to mimic clinical insight and provide thresholds for splitting "negative" and "equivocal" calls. Now, most calls were "positive" or "equivocal" (A1: 98.3, A2: 100%, A3: 99.6%), with just five false negatives remaining **(Figure 7).** Importantly, this strategy also resolves the discordance that was observed between assays **(Figure 7B),** as excluding equivocal calls now resulted in an overall concordance of 96% for VAFs ≤1% **(Figure 7C** and **Figure 8D).** This highlights how ctDNA assay discordance primarily arises when technical capabilities are unknowingly exceeded, which can be mitigated via our LOD-aware approach.

**Example 3: LOD predictions improve the reliability of clinical NGS tests**

The clinical performance of the LOD-aware approach was evaluated as follows.

***Materials and methods***

*Materials and methods as in Examples 1 and 2, and additionally:*

*Droplet digital PCR (ddPCR):* The QX100 ddPCR system from BioRad (ddPCR, Biorad, Hercules, CA, USA) was used, which combines a water-oil emulsion droplet technology with microfluidics (Biorad, 186-3005). All reactions were prepared using the ddPCR 2x Supermix for probes concentrated 2X (Biorad, 186-3024). The probes used targeting *EGFR* have previously been described *(Garcia et al., 2017, supra).* The ddPCR probes covered three mains somatic alterations of *EGFR*: various deletions of exon 19, p.L858R and p.T790M.

*Digital PCR BEAMing:* The EGFR p.T790M BEAMing assay was used, which is a highly sensitive and a quantitative digital PCR platform utilizing Beads, Emulsion, Amplification and Magnetics (BEAMing) provided by Sysmex Inostics (Hamburg, Germany, EU). This assay is based on a multiplex PCR targeting somatic alterations which are then followed by a massively parallel second PCR amplification performed on magnetic beads compartmentalized in millions of oil emulsions. Finally, a hybridization step with fluorescent probes specific to wild-type (WT) or mutant (MT) signals is performed by flow cytometry in order to discriminate these populations. The OncoBEAM-EGFR™ VI kit (Sysmex Inostics, Hamburg Germany) was used, enabling only the detection of p.T790M. All experiments were performed according to the supplier's IVD recommendations for clinical applications. The pre-specified positivity threshold for each codon was established in a clinical study of 186 patients, and the clinical cut-off was defined as 50 mutant beads detected and an alternative allelic fraction superior of >0.02%.

*Sensitivity analysis:* In order to estimate which factor was affecting the LOD the most for a given variant in a given clinical sample, a sensitivity analysis for assays A1 and A2 was performed. Since background noise, coverage depth and input material have different units and order of magnitudes, the impact of a relative change of 10% was evaluated for each parameter. More precisely, the change in the probability of detection $P_{detect}$ for such a parameter perturbation was calculated at the LOD. For example, for coverage $N$ the change in the probability of detection $\Delta P_N$ was given by:

$$\Delta P_N = P_{detect}(1.1 \times N, input, \theta) - P_{detect}(N, input, \theta)$$

$$(\text{Eq. 8})$$

*Results*

Having calibrated this LOD-aware approach using reference samples, its clinical performance was evaluated. NGS datasets from 580 NSCLC (non-small cell lung cancer) cfDNA samples were used, processed using assays A1 (n=272) and A2 (n=308). Three actionable mutations in *EGFR* (epidermal growth factor receptor gene) and one in *KRAS* (gene coding K-Ras protein) were selected, and digital PCR (dPCR, either droplet dPCR or BEAMing) used to confirm their status. This revealed 170 mutations across 126 patients, encompassing diverse VAFs and input amounts **(Figure 9A)**. Next, conventional variant calling was performed, and the results compared to those obtained by dPCR **(Figure 9B)**. It was observed that NGS assays A1 and A2 identified just 68% and 46% of the dPCR-confirmed variants, highlighting how false negatives are a major problem.

Then, it was tested whether LOD-aware variant calling solves this by disclosing potential false negatives. First, the accuracy of our model for clinical samples was confirmed, by comparing the confirmed and model predicted sensitivities for each variant **(Figure 10)**. Next, the standard variant calls were reclassified using our LOD-aware approach **(Figure 9C)**. A minimum VAF of interest of 1% was assumed, mimicking a clinical scenario in which VAFs of ≥1% are predictive of treatment response or have prognostic value and must be detected (Beau-Faller et al., 2020, Lung Cancer 140, 19-26). Using this threshold, negative calls with sufficient LODs (≤1%) were labelled "negative", whereas those with insufficient LODs (>1%) were reclassified as equivocal **(Figure 9D)**. In contrast to previous analyses, which only included cases where a confirmed variant was present, all measurements for the *KRAS* and three *EGFR* sites were plotted (i.e. confirmed positive and confirmed negative cases). Comparing our LOD-aware NGS results (excluding equivocal calls) to the dPCR "ground truth" revealed that only one false negative remained, improving inter-assay concordance from 94% to 99% for the dPCR-confirmed variants **(Figure 9D)**. It was concluded that LOD-aware variant calling accurately predicts the limitations of clinical ctDNA assays, foresees most false negatives and significantly improves reliability.

A unique advantage of this approach is that it is readily scalable, which was exploited to predict the ability of assays A1 and A2 to detect 89 clinically relevant variants in the NSCLC samples **(Figure 11)**. Here, the idea was to evaluate each site in a sample to establish whether a variant could be detected (low LOD) or was likely to be overlooked (high LOD). This uncovered a remarkably heterogenous "LOD landscape", with 100-fold variation between positions, samples and assays. This has reinforced the importance of reporting individual LODs for each sample and genomic positions, as enabled by our approach. Our approach also revealed the main technical factor that limits sensitivity at each position in each sample. Specifically, the change in sensitivity for a variant that is currently "just detected" (i.e. whose VAF equals the predicted LOD) was calculated for the scenario in which a model input (background noise, coverage depth or input amount) is adjusted by 10% **(Figure 11** and methods: *Sensitivity analysis*). This analysis was particularly informative for variants that had high (i.e. in most cases insufficient) LODs, and revealed that, for assay A1, increasing input amount led to the biggest improvement in sensitivity (and thus LOD). In contrast, increased sequencing coverage was most beneficial for assay A2.

In conclusion, our LOD-aware approach improves NGS-based variant calling by revealing LOD bottlenecks, predicting errors and increasing the transparency and reliability of variant reporting. Ultimately, this enables better informed clinical decisions. As all NGS assays are governed by the same biophysical principles, our framework is applicable to diverse applications.

Further advantages and benefits of the proposed methods

Applying the three-class, LOD-aware variant calling strategy to clinical samples demonstrated that it foresees most false negative calls and increases inter-assay concordance to ≥99%. The proposed approach is easily scalable, which was demonstrated by computing the "LOD landscape" for 580 non-small cell lung cancer (NSCLC) samples. This uncovered a 100-fold variation in LOD across samples and genomic positions and revealed which factors should be tuned to improve LOD. It may be concluded that high-resolution, adaptive LOD predictions improve the reliability and transparency of NGS testing, and could greatly benefit diverse applications including liquid biopsies, solid tumour analyses, personalised immunotherapy and biomarker discovery. The proposed methods and system frammeworks enable this concept to be easily applied, and may thus become a central part of clinical trials, technology development and clinical decision making.

A comprehensive model of the NGS workflow accurately predicts incorrect calls, and in doing so reveals a remarkable heterogeneous LOD landscape. Variability in LOD is driven by technical factors acting at assay, sample and nucleotide levels, explaining why reporting a single global LOD (currently standard practise) leads to false negatives *(Stetson et al., 2019, supra).* The proposed LOD-aware variant calling methods and systems confronts this heterogeneity head on by computing position-specific LODs, answering calls for better strategies to evaluate NGS assays and report their limitations (U.S. Food and Drug Administration, 13 April 2018*, supra; Merker et al., 2018, supra; Tack et al., 2018, supra).* As will be apparent to those skilled in the art, by assigning a third label ("equivocal") to unreliable variant calls, false results may be more easily foreseen and managed. This dramatically improves the reproducibility (96-99% concordance) and transparency of genetic testing, ultimately leading to more efficient and reliable clinical decisions.

As will be apparent to those skilled in the art, the proposed approach has many advantages. First, it distinguishes equivocal and confident negative calls, which reduces false negatives and enables retesting to focus on essential positions, avoiding unnecessary delays. NGS tests are increasingly relying on combining data from many loci to improve sensitivity, expand the range of assayable patients or indications *(Zviran et al., 2020, supra;* Wan et al., 2020, Sci. Transl. Med. 12, eaaz8084*; Mouliere et al., 2018, supra)* or compute an aggregate score (e.g. tumour mutational burden). The proposed approach reveals which positions should be included to maximise sensitivity and minimise false negatives, which otherwise dilute the biological signal. It also "rescues" samples with low overall quality by identifying positions with sufficient sensitivity, spots near miss positive calls where LOD is borderline, and helps optimise bioinformatic thresholds to minimise false positives while maintaining the required sensitivity. Clinical trials may thus benefit from the ability of our approach to standardise results from different study centres, patients or timepoints *(*Siravegna et al., 2015, Nat. Med. 21, 795-801*),* and technology development will be expedited by using an LOD-aware strategy to identify performance bottlenecks.

Other embodiments and applications

While various embodiments have been described above, it should be understood that they have been presented by way of example and not limitation. It will be apparent to persons skilled in the relevant art(s) that various changes in form and detail can be made therein without departing from the spirit and scope. In fact, after reading the above description, it will be apparent to one skilled in the relevant art(s) how to implement alternative embodiments.

While exemplary embodiments and applications of the proposed methods have been described in relation with the targeted next generation sequencing genomic analysis, it will be apparent to those skilled in the art of NGS analysis that they may also be adapted to incorporate additional features. The variant status triage could also be improved to account for the proportion of ctDNA in a cfDNA sample by using features extracted from NGS data such as DNA fragment size, nucleosome positions or mutation signatures *(Jovelet et al., 2016, supra;* Chabon et al., 2020, Nature 580, 245-251*;* Adalsteinsson et al., 2017, Nat. Commun. 8, 1324*).*

The Genomic Data Analyser 120 computer system (also "system" herein) 120 may be programmed or otherwise configured to implement different genomic data analysis methods in addition to the LOD-aware variant calling systems and methods as described herein, such as receiving and/or combining sequencing data, calling copy number alterations and/or annotating variants to further characterize the tumour samples.

The proposed LOD-aware framework was validated by experiments for one of the most challenging branches of precision medicine - variant calling from ctDNA - but as the generative model within this framework is based on universal biophysical principles, this approach can benefit many NGS applications. For example, it can be easily adapted for assays detecting gene fusions, copy number alterations, epigenetic changes or nucleosome remodelling, In the context of solid tumour biopsies it can address cellular heterogeneity by revealing the lower detection limit for rare cell subpopulations (e.g. those that have acquired resistance to a treatment). More generally, as NGS technologies increasingly strive towards earlier detection of disease onset, resistance and recurrence, they will be pushed to their limits and LOD aware approaches will become indispensable.

In summary, the proposed systems and methods pave the way to a new standard in variant calling and offers a much-needed improvement in the reliability of NGS-based clinical testing.

Unless otherwise defined, all technical and scientific terms used herein have the same meaning as commonly understood by one of ordinary skill in the art to which this invention belongs. The terminology used in the description herein is for describing particular embodiments only and is not intended to be limiting. As used in the description and the appended claims, the singular forms "a," "an," and "the" are intended to include the plural forms as well, unless the context clearly indicates otherwise.

Unless indicated to the contrary, the numerical parameters set forth in the following specification and attached claims are approximations that may vary depending upon the desired properties sought to be obtained and thus may be modified by the term "about". At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope are approximations, the numerical values set forth in the specific examples are reported as precisely as possible. Any numerical value, however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

As will be apparent to those skilled in the art of digital data communications, the methods described herein may be indifferently applied to various data structures such as data files or data streams. The terms "data", "data set", "data structures", "data fields", "file", or "stream" may thus be used indifferently throughout this specification.

Although the detailed description above contains many specific details, these should not be construed as limiting the scope of the embodiments but as merely providing illustrations of some of several embodiments.

In addition, it should be understood that any figures which highlight the functionality and advantages are presented for example purposes only. The disclosed methods are sufficiently flexible and configurable such that they may be utilized in ways other than that shown.

**Claims**

1. A method for estimating a limitation of detecting a nucleic acid sequence variant (*chr,pos,alt,ref)* in data generated by a next-generation-sequencing assay from a patient sample, the method comprising :

   - Obtaining alignment data, relative to a reference genome, from the patient sample NGS data;
   - Identifying from the alignment data, with a variant caller, that said variant does not have a positive call status;
   - Obtaining a measurement of the molecular count for the patient sample at the genomic position (*chr,pos*) of said variant;
   - Obtaining one or more analytical factors of the NGS assay used to process the patient sample;
   - Producing, with a statistical model, synthetic alignment data for one or more simulated VAFs as a function of the measured molecular count and the analytical factors of the NGS assay;
   - Estimating, from the synthetic alignment data, the detection sensitivity limitation of said variant caller as a function of one or more of the simulated VAFs, for said assay, said DNA sample and said variant *(chr, pos, ref, alt).*

2. The method of claim 1, further comprising:

   - Obtaining a user-defined minimal variant allele fraction of interest (mVAF) for said variant;
   - Obtaining a user-defined desired sensitivity for calling said variant as positive;
   - Estimating, from the estimated detection sensitivity function, the sensitivity for said mVAF;
   - Classifying the variant status as negative or equivocal as a function of said desired and estimated sensitivity.

3. The method of claim 1 or 2, further comprising:

   - Obtaining a user-defined desired sensitivity for calling said variant as positive;
   - Estimating, from the estimated detection sensitivity function, a limit of detection $LOD_{est}$ value as the lowest simulated VAF detectable with a sensitivity larger or equal to the user-defined sensitivity.

4. The method of claim 3, further comprising reporting the estimated limit of detection $LOD_{est}$ value.

5. The method of claim 1, further comprising:

   - Obtaining a user-defined minimal variant allele fraction of interest (mVAF) for said variant;
   - Obtaining a user-defined desired sensitivity for calling said variant as positive;
   - Estimating, from the estimated detection sensitivity function, a limit of detection $LOD_{est}$ value as the lowest simulated VAF detectable with a sensitivity larger or equal to the user-defined sensitivity.
   - If the mVAF for said variant is larger or equal to the $LOD_{est}$, classifying the variant status as negative, otherwise classifying the variant status as equivocal.

6. The method of claim 2 or 5, further comprising reporting the variant status.

7. The method of any preceding claims, wherein the NGS assay features a molecular identifier, further comprising estimating the molecular count in the NGS sequencing data according to the molecular barcoding measurements in the alignment data.

8. The method of any preceding claims, further comprising measuring, in the alignment data, the total coverage at the genomic position (*chr,pos*) of said variant and producing, with the statistical model, the synthetic alignment data for one or more simulated VAFs as a function of the coverage;

9. The method of any preceding claims, wherein the NGS assay does not feature a molecular identifier and the analytical factors of the NGS assay comprise a library conversion rate (LCR) profile, further comprising:

   - obtaining a DNA sample amount measurement;
   - estimating the molecular count in the library as a function of the DNA sample amount and the LCR value for said genomic position *(chr, pos)* in the LCR profile.

10. The method of claim 9, wherein the LCR profile is a constant value for all genomic positions or a table of the library conversion rate value at each genomic position or set of positions.

11. The method of claims 9 or 10 wherein the LCR profile depends on a DNA sample type.

12. The method of any preceding claims, wherein the analytical features of the NGS assay comprise an NGS assay error profile as a constant value for all variants, a table of the error rate value at each variant position *(chr,pos)* or set of positions, a table of the error rate value for each variant mutation type *(alt,ref)* or set of variant mutations, or a table of the error rate value for each variant (*chr,post,ref,alt*).

13. The method of claims 12, wherein the NGS workflow error profile depends on a DNA sample type.

14. The method of any of the claims 1 to 13, wherein producing, with a statistical model, synthetic sequencing data for different simulated VAFs comprises producing for each simulated VAF value one or more BAM files or different NGS data alignment features.

15. The method of any preceding claims, wherein the statistical model is a machine learning generative model or a biophysical generative model.

DNA samples

DNA library preparation ——— 100

DNA fragment

NGS Sequencer ——— 110

Laboratory

Raw NGS sequencing data

Sequence alignment ——— 121

Alignment data

Variant Caller

——— 120

Genomic Data Analyzer    {Positive (*chr,pos,ref,alt*)}

——— 123

Report

Figure 1 (prior art)

Figure 2

Figure 3

Figure 4

Figure 5

Figure 5 (continued)

Figure 6

Figure 6 (continued)

Figure 7

Standard variant calling workflow

Wet lab: Clinical cfDNA sample → Amplified library → Sequencing data

Variant caller

(iv) Positive call — Data support the variant

(iv) Negative call — Data enable variant to be ruled out

DNA amount (ii)

Coverage (each position) (ii)

Generative model (in silico): Series of VAFs (i) → Simulated ctDNA (i) → Amplified library (i) → Sequencing data (i) → $N_{ALT}$ / N

In silico data — Data from different VAFs

Sensitivity / Required sensitivity / VAF / Predicted LOD / 1.5

LOD prediction

(iii) LCR

Error rate (each position) (iii)

Initial setup: rsctDNA or confirmed samples → Initial NGS assay characterisation

LOD sufficient — Relevant VAFs

(iv) Equivocal (report LOD) — Insufficient evidence: variant might be present

LOD insufficient — Relevant VAFs

A

# Figure 7    B

117 confirmed variants
(13 variants x 3 dilutions x 3 DNA input amounts)

C

A

Simulated ctDNA

Sequencing data

VAF

Number of molecules M

Error rate

$M_{ALT}$
$M$

B

Figure 8

True positives

100
50
0

Observed sensitivity (%)

1.00
0.75
0.50
0.25
0.00

False negatives

100
50
0

0.00  0.25  0.50  0.75  1.00

Predicted sensitivity (%)

Figure 8 (continued)

Figure 9

**D**

Figure 9 (continued)

Figure 10

Figure 11

Europäisches
Patentamt

European
Patent Office

Office européen
des brevets

# EUROPEAN SEARCH REPORT

Application Number

EP 20 19 9741

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHANG XU ET AL: "Detecting very low allele fraction variants using targeted DNA sequencing and a novel molecular barcode-aware variant caller", BMC GENOMICS, BIOMED CENTRAL LTD, LONDON, UK, vol. 18, no. 1, 3 January 2017 (2017-01-03), pages 1-11, XP021265460, DOI: 10.1186/S12864-016-3425-4 * title, abstract, p.3, 4,6, 7, 9,10, table2 ,3, fig. 4,5 * | 1-8,14, 15 | INV. G16B20/20 G16B40/20 |
| X | & Xu Chang ET AL: "Supplementary Materials for: Detecting very low allele fraction variants using targeted DNA sequencing and a novel molecular barcode-aware variant caller", , 1 March 2017 (2017-03-01), XP055781218, Retrieved from the Internet: URL:https://static-content.springer.com/esm/art:10.1186/s12864-016-3425-4/MediaObjects/12864_2016_3425_MOESM2_ESM.pdf [retrieved on 2021-03-02] * p.4 * | 8 | |
| A | XU CHANG: "A review of somatic single nucleotide variant calling algorithms for next-generation sequencing data", COMPUTATIONAL AND STRUCTURAL BIOTECHNOLOGY JOURNAL, vol. 16, 1 January 2018 (2018-01-01), pages 15-24, XP055781134, Sweden ISSN: 2001-0370, DOI: 10.1016/j.csbj.2018.01.003 * the whole document * | 1-15 | |

TECHNICAL FIELDS SEARCHED (IPC)

G16B

-/--

The present search report has been drawn up for all claims

| Place of search | Date of completion of the search | Examiner |
|---|---|---|
| Munich | 2 March 2021 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 19 9741

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/237838 A1 (SAKARYA ONUR [US] ET AL) 23 August 2018 (2018-08-23) * the whole document * ----- | 1-15 | |

TECHNICAL FIELDS
SEARCHED        (IPC)

The present search report has been drawn up for all claims

| Place of search | Date of completion of the search | Examiner |
|---|---|---|
| Munich | 2 March 2021 | Lüdemann, Susanna |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 20 19 9741

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2021

| Patent document cited in search report | Publication date | Patent family member(s) | Publication date |
|---|---|---|---|
| US 2018237838 A1 | 23-08-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017220508 A
- EP 2020076246 W
- US 50674626 B

**Non-patent literature cited in the description**

- **CHUAH ; CHEW.** *J. Immunother. cancer,* 2020, vol. 8, e000363
- **ZVIRAN et al.** *Nat. Med.,* 2020, vol. 26 (7), 1114-1124
- **MALONE et al.** *Genome Med.,* 2020, vol. 12, 8
- **STETSON et al.** *JCO Precis. Oncol.,* 2019, 1-9
- **PAWELETZ et al.** *JCO Precis. Oncol.,* 2019, vol. 1-3
- **KUDERER et al.** *JAMA Oncol.,* 2017, vol. 3, 996-998
- **KIM et al.** *PLoS One,* 2019, vol. 14, e0222535
- **HEITZER et al.** *Nat. Rev. Genet.,* 2019, 20
- **MATSUMOTO et al.** *Lung Cancer,* 2020, vol. 139, 80-88
- **JIANG et al.** *Mol Med Rep,* 2019, vol. 20, 593-603
- **HEITZER et al.** *Trends Mol. Med.,* 2020, vol. 26, 519-528
- **SUN et al.** *PNAS,* 2015, vol. 112, E5503 LP-E5512
- **CHIEN et al.** *J. Clin. Oncol.,* 2017, vol. 35, e23065-e23065
- **PLAGNOL et al.** *PLoS One,* 2018, vol. 13, e0193802
- **NEWMAN et al.** *Nat. Biotechnol.,* 2016, vol. 34, 547-555
- *U.S. Food and Drug Administration,* 13 April 2018
- **MERKER et al.** *J. Clin. Oncol.,* 2018, vol. 36, 1631-1641
- **TACK et al.** *J. Mol. Diagnostics,* 2018, vol. 20, 743-753
- **BLOMQUIST et al.** *Biomol. Detect. Quantif.,* 2015, vol. 5, 30-37
- **PETRACKOVA et al.** *Front. Oncol.,* 2019, vol. 9, 1-6
- **MA et al.** *Genome Biol.,* 2019, vol. 20, 50
- **XU et al.** *BMC Genomics,* 2017, vol. 18, 5
- **XU et al.** A review of somatic single nucleotide variant calling algorithms for next-generation sequencing data. *Computational and Structural Biotechnology Journal,* February 2018, vol. 16, 15-24
- **SHUGAY et al.** MAGERI: Computational pipeline for molecular-barcoded targeted resequencing. *PLoS Comput. Biol.,* May 2017, vol. 13, 5
- **XU et al.** smCounter2: an accurate low-frequency variant caller for targeted sequencing data with unique molecular identifiers. *Bioinformatics,* April 2019, vol. 35, 8
- **GARCIA et al.** *Oncotarget,* 2017, vol. 8 (50
- **GARCIA et al.** *Oncotarget,* 2018, vol. 9 (30
- **LI ; DURBIN.** *Bioinformatics,* 2009, vol. 25, 1754-1760
- **SCHMITT et al.** *PNAS,* 2012, vol. 109, 14508 LP-14513
- **RAMU et al.** *Nat. Methods,* 2013, vol. 10, 985-987
- **GEEURICKX ; HENDRIX.** *Mol. Aspects Med.,* 2020, vol. 72, 100828
- **SNYDER et al.** *Cell,* 2016, vol. 164, 57-68
- **MOULIERE et al.** *Sci. Transl. Med.,* 2018, vol. 10, eaat4921
- **JOVELET et al.** *Clin. Cancer Res.,* 2016, vol. 22, 2960 LP-2968
- **DAVIS ; RABINOWITZ.** Methods of Numerical Integration. Dover Publications, 2007
- **BEAU-FALLER et al.** *Lung Cancer,* 2020, vol. 140, 19-26
- **WAN et al.** *Sci. Transl. Med.,* 2020, vol. 12, eaaz8084
- **SIRAVEGNA et al.** *Nat. Med.,* 2015, vol. 21, 795-801
- **CHABON et al.** *Nature,* 2020, vol. 580, 245-251
- **ADALSTEINSSON et al.** *Nat. Commun.,* 2017, vol. 8, 1324